# EUROPEAN PATENT APPLICATION

(11) **EP 4 406 552 A1**
(43) Date of publication of application: **31.07.2024**
(21) Application number: 22872175.9
(22) Date of filing: 26.09.2022
(51) Int. Cl.: A61K 39/395, A61P 35/00, A61K 31/4725

(54) **USE OF ANTI-CD40 ANTIBODY**

(30) Priority: 26.09.2021 CN 202111127584
(71) Applicant: CHIA TAI TIANQING PHARMACEUTICAL GROUP CO., LTD., Lianyungang, Jiangsu 222062 (CN)
(72) Inventor: SHEN, Chen, Nanjing, Jiangsu 211100 (CN); LV, Peng, Nanjing, Jiangsu 211100 (CN); TIAN, Xin, Nanjing, Jiangsu 211100 (CN); YANG, Ling, Nanjing, Jiangsu 211100 (CN); ZHANG, Xiquan, Nanjing, Jiangsu 211100 (CN)
(74) Representative: WSL Patentanwälte Partnerschaft mbB
(86) International application number: PCT/CN2022/121184
(87) International publication number: WO 2023/046131

(57) **Abstract**

Provided is the use of an anti-CD40 antibody, specifically, provided is a combined drug for treating cancer. The combined drug comprises an anti-CD40 antibody or an antigen-binding portion thereof and a tyrosine kinase inhibitor, and has a good anti-tumor activity and safety.

## Description

### TECHNICAL FIELD

The present application relates to the field of biopharmaceuticals, and particularly, to a pharmaceutical combination for treating cancers.

### BACKGROUND

CD40 is a member of the tumor necrosis factor receptor (TNFR) superfamily. It is present on antigen-presenting cells (APCs) such as B cells, dendritic cells (DCs), macrophages and the like, and is also expressed on other activated hematopoietic cells (e.g., T cells). Furthermore, CD40 is expressed on a wide range of tumor cells, for example, cells of B-cell tumors (non-Hodgkin's lymphoma, acute lymphocytic leukemia, chronic lymphocytic leukemia, myeloma, etc.) and epithelial tumors (prostate cancer, liver cancer, lung cancer, breast cancer, etc.) and etc.

The natural ligand of CD40 (known as CD154 or CD40L) is mainly expressed on activated T lymphocytes and mast cells. The CD40/CD40L signaling pathway is involved in humoral immunity and cellular immune response of the body, and plays a key regulatory role in T cell-dependent antibody immune response and inflammatory response, such as the activation, proliferation and differentiation of B cells, production of antibodies, class conversion of antibodies, and the like. Thus, the treatment with CD40 agonists (e.g., agonistic anti-CD40 antibodies) may enhance the anti-tumor immune response of the body.

Tyrosine kinases are a group of enzymes that catalyze the phosphorylation of tyrosine residues in proteins. It plays an important role in intracellular signal transduction, takes part in the regulation, signaling and development of normal cells, and is closely related to the proliferation, differentiation, migration and apoptosis of tumor cells. Many receptor tyrosine kinases are associated with tumorigenesis and can be classified into epidermal growth factor receptors (EGFRs), platelet-derived growth factor receptors (PDGFRs), vascular endothelial growth factor receptors (VEGFRs), fibroblast growth factor receptors (FGFRs) and the like according to the structure of the extracellular domain.

Anlotinib is a quinoline derivative tyrosine kinase inhibitor and plays a role in influencing tumor angiogenesis and proliferation signal transduction as a multi-target tyrosine kinase inhibitor (TKI). Its major targets include: receptor tyrosine kinase vascular endothelial growth factor receptor (VEGFR) 1-3, epidermal growth factor receptors (EGFRs), fibroblast growth factor receptor (FGFR) 1-4, platelet-derived growth factor receptor (PDGFR) α and β, and stem cell factor receptor (SCFR) 7, 8, and 9.

Although any of the existing treatments may be beneficial to the patients, there is still a need for more therapies with reduced toxicity and improved outcomes for clinical use.

### SUMMARY

In a first aspect, the present application provides a pharmaceutical combination, comprising: (a) an anti-CD40 antibody or an antigen-binding portion thereof, and (b) a second therapeutic agent. In some embodiments, the second therapeutic agent has therapeutic efficacy against a cancer. In some embodiments, the second therapeutic agent includes tyrosine kinase inhibitors. In some embodiments, the second therapeutic agent includes VEGFR tyrosine kinase inhibitors. In some specific embodiments, the second therapeutic agent includes anlotinib or a pharmaceutically acceptable salt thereof.

The tyrosine kinase inhibitors refer to any substances or reagents capable of inhibiting (e.g., blocking, disrupting, or inactivating) tyrosine kinase or downstream signal transduction of tyrosine kinases, including micromolecular tyrosine kinase inhibitors and antibodies. The tyrosine kinase inhibitors include receptor tyrosine kinase inhibitors and non-receptor tyrosine kinase inhibitors. In some embodiments, the receptor tyrosine kinase inhibitors include, but are not limited to, EGFR tyrosine kinase inhibitors, VEGFR tyrosine kinase inhibitors, FGFR tyrosine kinase inhibitors, SCFR tyrosine kinase inhibitors, PDGFR tyrosine kinase inhibitors, and the like.

The VEGFR tyrosine kinase inhibitors refer to any substances or reagents that inhibit (e.g., block, disrupt, or inactivate) VEGF receptor tyrosine kinases or downstream signal transduction of VEGF receptor tyrosine kinases, including micromolecular VEGFR tyrosine kinase inhibitors and antibodies. Examples of the VEGFR tyrosine kinase inhibitor include, but are not limited to, anlotinib, vandetanib, sorafenib, cediranib, vatalanib, pazopanib, motesanib, lenvatinib, sunitinib, bevacizumab, ramucirumab, or the pharmaceutically acceptable salt thereof, or any combination of the foregoing.

Optionally, the pharmaceutical combination further comprises a third therapeutic agent having therapeutic efficacy against a cancer. In some embodiments, the third therapeutic agent can be therapeutic agents against cancer known in the art, such as chemotherapeutic drugs, radiotherapeutic drugs, surgery, or the combination thereof.

In some embodiments, the pharmaceutical combination comprises: (a) the anti-CD40 antibody or the antigen-binding portion thereof, (b) the tyrosine kinase inhibitor, and optionally, (c) the chemotherapeutic drug. In some embodiments, the pharmaceutical combination comprises: (a) the anti-CD40 antibody or the antigen-binding portion thereof, (b) the VEGFR tyrosine kinase inhibitor, and optionally, (c) the chemotherapeutic drug. In some specific embodiments, the pharmaceutical combination comprises: (a) the anti-CD40 antibody or the antigen-binding portion thereof, (b) anlotinib or the pharmaceutically acceptable salt thereof, and optionally, (c) the chemotherapeutic drug.

In some embodiments, the pharmaceutical combination comprises: (a) a formulation of the anti-CD40 antibody or the antigen-binding portion thereof, (b) a formulation of the tyrosine kinase inhibitor, and optionally, (c) the chemotherapeutic drug. In some embodiments, the pharmaceutical combination comprises: (a) a formulation of the anti-CD40 antibody or the antigen-binding portion thereof, (b) a formulation of the VEGFR tyrosine kinase inhibitor, and optionally, (c) the chemotherapeutic drug. In some specific embodiments, the pharmaceutical combination comprises: (a) a formulation of the anti-CD40 antibody or the antigen-binding portion thereof, (b) a formulation of anlotinib or the pharmaceutically acceptable salt thereof, and optionally, (c) the chemotherapeutic drug.

In some embodiments, the pharmaceutical combination is packaged in the same kit further comprising an instruction for combined use of the anti-CD40 antibody or the antigen-binding portion thereof and the tyrosine kinase inhibitor in treating a cancer. In some embodiments, the anti-CD40 antibody or the antigen-binding portion thereof and the tyrosine kinase inhibitor in the pharmaceutical combination are packaged separately in their respective kits further comprising an instruction for combined use of the anti-CD40 antibody or the antigen-binding portion thereof and the tyrosine kinase inhibitor in treating a cancer.

In some embodiments, the pharmaceutical combination is packaged in the same kit further comprising an instruction for combined use of the anti-CD40 antibody or the antigen-binding portion thereof, the tyrosine kinase inhibitor, and the chemotherapeutic drug in treating a cancer. In some embodiments, the anti-CD40 antibody or the antigen-binding portion thereof, the tyrosine kinase inhibitor, and the chemotherapeutic drug in the pharmaceutical combination are packaged separately in their respective kits further comprising an instruction for combined use of the anti-CD40 antibody or the antigen-binding portion thereof, the tyrosine kinase inhibitor, and the chemotherapeutic drug in treating a cancer.

In some embodiments, the pharmaceutical combination is packaged in the same kit further comprising an instruction for combined use of the anti-CD40 antibody or the antigen-binding portion thereof and the VEGFR tyrosine kinase inhibitor in treating a cancer. In some embodiments, the anti-CD40 antibody or the antigen-binding portion thereof and the VEGFR tyrosine kinase inhibitor in the pharmaceutical combination are packaged separately in their respective kits further comprising an instruction for combined use of the anti-CD40 antibody or the antigen-binding portion thereof and the VEGFR tyrosine kinase inhibitor in treating a cancer.

In some embodiments, the pharmaceutical combination is packaged in the same kit further comprising an instruction for combined use of the anti-CD40 antibody or the antigen-binding portion thereof, the VEGFR tyrosine kinase inhibitor, and the chemotherapeutic drug in treating a cancer. In some embodiments, the anti-CD40 antibody or the antigen-binding portion thereof, the VEGFR tyrosine kinase inhibitor, and the chemotherapeutic drug in the pharmaceutical combination are packaged separately in their respective kits further comprising an instruction for combined use of the anti-CD40 antibody or the antigen-binding portion thereof, the VEGFR tyrosine kinase inhibitor, and the chemotherapeutic drug in treating a cancer.

In some embodiments, the pharmaceutical combination is packaged in the same kit further comprising an instruction for combined use of the anti-CD40 antibody or the antigen-binding portion thereof and anlotinib or the pharmaceutically acceptable salt thereof in treating a cancer. In some embodiments, the anti-CD40 antibody or the antigen-binding portion thereof and anlotinib or the pharmaceutically acceptable salt thereof in the pharmaceutical combination are packaged separately in their respective kits further comprising an instruction for combined use of the anti-CD40 antibody or the antigen-binding portion thereof and anlotinib or the pharmaceutically acceptable salt thereof in treating a cancer.

In some embodiments, the pharmaceutical combination is packaged in the same kit further comprising an instruction for combined use of the anti-CD40 antibody or the antigen-binding portion thereof, anlotinib or the pharmaceutically acceptable salt thereof, and the chemotherapeutic drug in treating a cancer. In some embodiments, the anti-CD40 antibody or the antigen-binding portion thereof, anlotinib or the pharmaceutically acceptable salt thereof, and the chemotherapeutic drug in the pharmaceutical combination are packaged separately in their respective kits further comprising an instruction for combined use of the anti-CD40 antibody or the antigen-binding portion thereof, anlotinib or the pharmaceutically acceptable salt thereof, and the chemotherapeutic drug in treating a cancer.

In some embodiments, the pharmaceutical combination is a fixed combination. In some embodiments, the fixed combination is present in the form of a solid formulation or a liquid formulation.

In some embodiments, the pharmaceutical combination is a non-fixed combination.

In some embodiments, the anti-CD40 antibody or the antigen-binding portion thereof and the tyrosine kinase inhibitor in the non-fixed combination are each present in the form of a formulation. In some embodiments, the anti-CD40 antibody or the antigen-binding portion thereof, the tyrosine kinase inhibitor, and the chemotherapeutic drug in the non-fixed combination are each present in the form of a formulation.

In some embodiments, the anti-CD40 antibody or the antigen-binding portion thereof and the VEGFR tyrosine kinase inhibitor in the non-fixed combination are each present in the form of a formulation. In some embodiments, the anti-CD40 antibody or the antigen-binding portion thereof, the VEGFR tyrosine kinase inhibitor, and the chemotherapeutic drug in the non-fixed combination are each present in the form of a formulation.

In some embodiments, the anti-CD40 antibody or the antigen-binding portion thereof and anlotinib or the pharmaceutically acceptable salt thereof in the non-fixed combination are each present in the form of a formulation. In some specific embodiments, the anti-CD40 antibody or the antigen-binding portion thereof in the non-fixed combination is present in the form of a liquid formulation, and anlotinib or the pharmaceutically acceptable salt thereof in the non-fixed combination is present in the form of a solid formulation. In some specific embodiments, the anti-CD40 antibody or the antigen-binding portion thereof in the non-fixed combination is present in the form of an injection, and anlotinib or the pharmaceutically acceptable salt thereof in the non-fixed combination is present in the form of an oral solid formulation. In some specific embodiments, the anti-CD40 antibody or the antigen-binding portion thereof in the non-fixed combination is present in the form of an injection, and anlotinib or the pharmaceutically acceptable salt thereof in the non-fixed combination is present in the form of a capsule. In some embodiments, the anti-CD40 antibody or the antigen-binding portion thereof, anlotinib or the pharmaceutically acceptable salt thereof, and the chemotherapeutic drug in the non-fixed combination are each present in the form of a formulation.

In a second aspect, the present application further provides use of the pharmaceutical combination in the first aspect for preparing a medicament for treating a cancer. In addition, the present application further provides use of the anti-CD40 antibody or the antigen-binding portion thereof and the tyrosine kinase inhibitor for preparing a medicament for treating a cancer. In addition, the present application further provides use of the anti-CD40 antibody or the antigen-binding portion thereof, the tyrosine kinase inhibitor, and the chemotherapeutic drug for preparing a medicament for treating a cancer. In addition, the present application further provides use of the anti-CD40 antibody or the antigen-binding portion thereof and the VEGFR tyrosine kinase inhibitor for preparing a medicament for treating a cancer. In addition, the present application further provides use of the anti-CD40 antibody or the antigen-binding portion thereof, the VEGFR tyrosine kinase inhibitor, and the chemotherapeutic drug for preparing a medicament for treating a cancer. In addition, the present application further provides use of the anti-CD40 antibody or the antigen-binding portion thereof and anlotinib or the pharmaceutically acceptable salt thereof for preparing a medicament for treating a cancer. In addition, the present application further provides use of the anti-CD40 antibody or the antigen-binding portion thereof, anlotinib or the pharmaceutically acceptable salt thereof, and the chemotherapeutic drug for preparing a medicament for treating a cancer.

In a third aspect, the present application further provides a method for treating a cancer in a subject, comprising administering to the subject an effective amount of the pharmaceutical combination in the first aspect. In some embodiments, the present application provides a method for treating a cancer in a subject, comprising administering to the subject an effective amount of the anti-CD40 antibody or the antigen-binding portion thereof, an effective amount of the tyrosine kinase inhibitor, and optionally, an effective amount of the chemotherapeutic drug. In some embodiments, the present application provides a method for treating a cancer in a subject, comprising administering to the subject an effective amount of the anti-CD40 antibody or the antigen-binding portion thereof, an effective amount of the VEGFR tyrosine kinase inhibitor, and optionally, an effective amount of the chemotherapeutic drug. In some embodiments, the present application provides a method for treating a cancer in a subject, comprising administering to the subject an effective amount of the anti-CD40 antibody or the antigen-binding portion thereof, an effective amount of anlotinib or the pharmaceutically acceptable salt thereof, and optionally, an effective amount of the chemotherapeutic drug.

In a fourth aspect, the present application provides a kit, comprising the pharmaceutical combination in the first aspect. In some embodiments, the kit comprises (a) a first formulation, comprising the anti-CD40 antibody or the antigen-binding portion thereof as the active ingredient; (b) a second formulation, comprising the tyrosine kinase inhibitor as the active ingredient; and optionally, (c) the chemotherapeutic drug. In some embodiments, the kit comprises (a) a first formulation, comprising the anti-CD40 antibody or the antigen-binding portion thereof as the active ingredient; (b) a second formulation, comprising the VEGFR tyrosine kinase inhibitor as the active ingredient; and optionally, (c) the chemotherapeutic drug. In some embodiments, the kit comprises (a) a first formulation, comprising the anti-CD40 antibody or the antigen-binding portion thereof as the active ingredient; (b) a second formulation, comprising anlotinib or the pharmaceutically acceptable salt thereof as the active ingredient; and optionally, (c) the chemotherapeutic drug. In some embodiments, the kit is used for treating a cancer.

In a fifth aspect, the present application further provides a method for treating a cancer, comprising administering to a subject an effective amount of the anti-CD40 antibody or the antigen-binding portion thereof of the present application. The present application further provides use of the anti-CD40 antibody or the antigen-binding portion thereof of the present application for treating a cancer. The present application further provides use of the anti-CD40 antibody or the antigen-binding portion thereof of the present application for preparing a medicament for treating a cancer.

### Administration/Treatment Regimen of Pharmaceutical Combination

In some embodiments, in the use or method described above, the anti-CD40 antibody or the antigen-binding portion thereof and the tyrosine kinase inhibitor are each present in the form of a formulation, and can be administered simultaneously, sequentially, and/or alternately. In some embodiments, in the use or method described above, the anti-CD40 antibody or the antigen-binding portion thereof, the tyrosine kinase inhibitor, and the chemotherapeutic drug are each present in the form of a formulation, and can be administered simultaneously, sequentially, and/or alternately. In some embodiments, in the use or method described above, the anti-CD40 antibody or the antigen-binding portion thereof and the VEGFR tyrosine kinase inhibitor are each present in the form of a formulation, and can be administered simultaneously, sequentially, and/or alternately. In some embodiments, in the use or method described above, the anti-CD40 antibody or the antigen-binding portion thereof, the VEGFR tyrosine kinase inhibitor, and the chemotherapeutic drug are each present in the form of a formulation, and can be administered simultaneously, sequentially, and/or alternately. In some embodiments, in the use or method described above, the anti-CD40 antibody or the antigen-binding portion thereof and anlotinib or the pharmaceutically acceptable salt thereof are each present in the form of a formulation, and can be administered simultaneously, sequentially, and/or alternately. In some embodiments, in the use or method described above, the anti-CD40 antibody or the antigen-binding portion thereof, anlotinib or the pharmaceutically acceptable salt thereof, and the chemotherapeutic drug are each present in the form of a formulation, and can be administered simultaneously, sequentially, and/or alternately.

In some embodiments, in the use or method described above, the anti-CD40 antibody or the antigen-binding portion thereof and the tyrosine kinase inhibitor are each present in the form of a formulation, and each is administered in an interval administration manner. In some embodiments, in the use or method described above, the anti-CD40 antibody or the antigen-binding portion thereof, the tyrosine kinase inhibitor, and the chemotherapeutic drug are each present in the form of a formulation, and each is administered in an interval administration manner. In some embodiments, in the use or method described above, the anti-CD40 antibody or the antigen-binding portion thereof and the VEGFR tyrosine kinase inhibitor are each present in the form of a formulation, and each is administered in an interval administration manner. In some embodiments, in the use or method described above, the anti-CD40 antibody or the antigen-binding portion thereof, the VEGFR tyrosine kinase inhibitor, and the chemotherapeutic drug are each present in the form of a formulation, and each is administered in an interval administration manner. In some embodiments, in the use or method described above, the anti-CD40 antibody or the antigen-binding portion thereof and anlotinib or the pharmaceutically acceptable salt thereof are each present in the form of a formulation, and each is administered in an interval administration manner. In some embodiments, in the use or method described above, the anti-CD40 antibody or the antigen-binding portion thereof, anlotinib or the pharmaceutically acceptable salt thereof, and the chemotherapeutic drug are each present in the form of a formulation, and each is administered in an interval administration manner. The interval administration comprises a administration period and a off-drug period, and the administration can be done once or multiple times daily during the administration period. For example, the treatment is administered daily in a treatment period, and then stopped for a period of time during the off-drug period, followed by another treatment period, and then off-drug period, which can be repeated multiple times.

In some embodiments, in the use or method described above, the anti-CD40 antibody or the antigen-binding portion thereof and the tyrosine kinase inhibitor are administered separately in the same or different administration regimens. In some embodiments, in the use or method described above, the anti-CD40 antibody or the antigen-binding portion thereof and the tyrosine kinase inhibitor are administered separately in the different administration regimen. In some embodiments, in the use or method described above, the anti-CD40 antibody or the antigen-binding portion thereof, the tyrosine kinase inhibitor, and the chemotherapeutic drug are administered separately in the same or different administration regimens. In some embodiments, in the use or method described above, the anti-CD40 antibody or the antigen-binding portion thereof, the tyrosine kinase inhibitor, and the chemotherapeutic drug are administered separately in the different administration regimen. In some embodiments, in the use or method described above, the anti-CD40 antibody or the antigen-binding portion thereof and the VEGFR tyrosine kinase inhibitor are administered separately in the same or different administration regimens. In some embodiments, in the use or method described above, the anti-CD40 antibody or the antigen-binding portion thereof and the VEGFR tyrosine kinase inhibitor are administered separately in the different regimen. In some embodiments, in the use or method described above, the anti-CD40 antibody or the antigen-binding portion thereof, the VEGFR tyrosine kinase inhibitor, and the chemotherapeutic drug are administered separately in the same or different administration regimens. In some embodiments, in the use or method described above, the anti-CD40 antibody or the antigen-binding portion thereof, the VEGFR tyrosine kinase inhibitor, and the chemotherapeutic drug are administered separately in the different regimen.

In some embodiments, in the use or method described above, the anti-CD40 antibody or the antigen-binding portion thereof and anlotinib or the pharmaceutically acceptable salt thereof are administered separately in the same or different administration regimens. In some embodiments, in the use or method described above, the anti-CD40 antibody or the antigen-binding portion thereof and anlotinib or the pharmaceutically acceptable salt thereof are administered separately in the different administration regimen. In some embodiments, in the use or method described above, the anti-CD40 antibody or the antigen-binding portion thereof, anlotinib or the pharmaceutically acceptable salt thereof, and the chemotherapeutic drug are administered separately in the same or different administration regimens. In some embodiments, in the use or method described above, the anti-CD40 antibody or the antigen-binding portion thereof, anlotinib or the pharmaceutically acceptable salt thereof, and the chemotherapeutic drug are administered separately in different administration regimen.

In some embodiments, in the use or method described above, the anti-CD40 antibody or the antigen-binding portion thereof can be administered once every week (q1w), once every 2 weeks (q2w), once every 3 weeks (q3w), or once every 4 weeks (q4w). In some embodiments, in the use or method described above, the anti-CD40 antibody or the antigen-binding portion thereof can be administered once every week (±3 days), once every 2 weeks (±3 days), once every 3 weeks (±3 days), or once every 4 weeks (±3 days). In one specific embodiment, the anti-CD40 antibody or the antigen-binding portion thereof can be administered once every 3 weeks (±3 days).

In some embodiments, in the use or method described above, the anti-CD40 antibody or the antigen-binding portion thereof is administered at a dose of 0.001 to 100 mg/kg body weight.

In some embodiments, in the use or method described above, the anti-CD40 antibody or the antigen-binding portion thereof is administered at a flat dose effective in treating the cancer. In some specific embodiments, the flat dose ranges from about 1 mg to about 1000 mg of the anti-CD40 antibody or the antigen-binding portion thereof.

In some embodiments, in the use or method described above, anlotinib or the pharmaceutically acceptable salt thereof is administered in treatment cycles of every 1 week, every 2 weeks, every 3 weeks, or every 4 weeks.

In some embodiments, in the use or method described above, anlotinib or the pharmaceutically acceptable salt thereof is administered in treatment cycles of every 1 week (±3 days), every 2 weeks (±3 days), every 3 weeks (±3 days), or every 4 weeks (±3 days). In one specific embodiment, anlotinib or the pharmaceutically acceptable salt thereof is administered in treatment cycles of every 3 weeks (±3 days).

In some embodiments, in the use or method described above, anlotinib or the pharmaceutically acceptable salt thereof can be administered intermittently, for example, consecutively 2-week treatment and then 2-week interruption, consecutively 2-week treatment and then 1-week interruption, or consecutively 5-day treatment and then 2-day interruption. The intermittent treatment cycle may be repeated multiple times.

In some embodiments, in the use or method described above, anlotinib or the pharmaceutically acceptable salt thereof is administered at a daily dose of 3 mg to 30 mg. In some embodiments, anlotinib or the pharmaceutically acceptable salt thereof is administered at a daily dose of 5 mg to 20 mg. In some embodiments, anlotinib or the pharmaceutically acceptable salt thereof is administered at a daily dose of 8 mg to 16 mg. In some embodiments, anlotinib or the pharmaceutically acceptable salt thereof is administered at a daily dose of 3 mg, 4 mg, 5 mg, 6 mg, 7 mg, 8 mg, 9 mg, 10 mg, 11 mg, 12 mg, 13 mg, 14 mg, 15 mg, and 16 mg. In one specific embodiment, anlotinib or the pharmaceutically acceptable salt thereof is administered at a daily dose of 8 mg. In one specific embodiment, anlotinib or the pharmaceutically acceptable salt thereof is administered at a daily dose of 10 mg. In one specific embodiment, anlotinib or the pharmaceutically acceptable salt thereof is administered at a daily dose of 12 mg. In some embodiments, anlotinib or the pharmaceutically acceptable salt thereof can be administered once or multiple times daily. In some embodiments, anlotinib or the pharmaceutically acceptable salt thereof can be administered once daily. In some embodiments, anlotinib or the pharmaceutically acceptable salt thereof is administered once daily in the form of an oral solid formulation.

In some specific embodiments, in the use or method described above, anlotinib or the pharmaceutically acceptable salt thereof can be administered once daily at a dose of 6 mg, 8 mg, 10 mg, or 12 mg on a regimen of 2-week consecutive treatment and then 1-week interruption. In some specific embodiments, in the use or method described above, anlotinib or the pharmaceutically acceptable salt thereof can be administered once daily at a dose of 6 mg, 8 mg, 10 mg, or 12 mg on a regimen of 2-week consecutive treatment and then 2-week interruption.

In some embodiments, in the use or method described above, the anti-CD40 antibody or the antigen-binding portion thereof and the tyrosine kinase inhibitor are administered separately in the same or different treatment cycles. In some embodiments, in the use or method described above, the anti-CD40 antibody or the antigen-binding portion thereof and the tyrosine kinase inhibitor are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof and the tyrosine kinase inhibitor are administered in treatment cycles of every 3 weeks (±3 days). In some embodiments, in the use or method described above, the anti-CD40 antibody or the antigen-binding portion thereof, the tyrosine kinase inhibitor, and the chemotherapeutic drug are administered separately in the same or different treatment cycles. In some embodiments, in the use or method described above, the anti-CD40 antibody or the antigen-binding portion thereof, the tyrosine kinase inhibitor, and the chemotherapeutic drug are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof, the tyrosine kinase inhibitor, and the chemotherapeutic drug are administered in treatment cycles of every 3 weeks (±3 days).

In some embodiments, in the use or method described above, the anti-CD40 antibody or the antigen-binding portion thereof and the VEGFR tyrosine kinase inhibitor are administered separately in the same or different treatment cycles. In some embodiments, in the use or method described above, the anti-CD40 antibody or the antigen-binding portion thereof and the VEGFR tyrosine kinase inhibitor are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof and the VEGFR tyrosine kinase inhibitor are administered in treatment cycles of every 3 weeks (±3 days). In some embodiments, in the use or method described above, the anti-CD40 antibody or the antigen-binding portion thereof, the VEGFR tyrosine kinase inhibitor, and the chemotherapeutic drug are administered separately in the same or different treatment cycles. In some embodiments, in the use or method described above, the anti-CD40 antibody or the antigen-binding portion thereof, the VEGFR tyrosine kinase inhibitor, and the chemotherapeutic drug are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof, the VEGFR tyrosine kinase inhibitor, and the chemotherapeutic drug are administered in treatment cycles of every 3 weeks (±3 days).

In some embodiments, in the use or method described above, the anti-CD40 antibody or the antigen-binding portion thereof and anlotinib or the pharmaceutically acceptable salt thereof are administered separately in the same or different treatment cycles. In some embodiments, in the use or method described above, the anti-CD40 antibody or the antigen-binding portion thereof and anlotinib or the pharmaceutically acceptable salt thereof are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof and anlotinib or the pharmaceutically acceptable salt thereof are administered in treatment cycles of every 3 weeks (±3 days). In some embodiments, in the use or method described above, the anti-CD40 antibody or the antigen-binding portion thereof, anlotinib or the pharmaceutically acceptable salt thereof, and the chemotherapeutic drug are administered separately in the same or different treatment cycles. In some embodiments, in the use or method described above, the anti-CD40 antibody or the antigen-binding portion thereof, anlotinib or the pharmaceutically acceptable salt thereof, and the chemotherapeutic drug are administered in the same treatment cycles, for example, in treatment cycles of every 1 week (±3 days), every 2 weeks (±3 days), every 3 weeks (±3 days), or every 4 weeks (±3 days). In one specific embodiment, the anti-CD40 antibody or the antigen-binding portion thereof, anlotinib or the pharmaceutically acceptable salt thereof, and the chemotherapeutic drug are administered in treatment cycles of every 3 weeks (±3 days).

### Anti-CD40 Antibody or Antigen-Binding Portion Thereof

As used herein, the anti-CD40 antibody or the antigen-binding portion thereof comprises:
i) a heavy chain variable region, comprising a V_{H} CDR1, a V_{H} CDR2, and a V_{H} CDR3, wherein (1) the V_{H} CDR1, V_{H} CDR2, and V_{H} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 4, and 7, respectively; (2) the V_{H} CDR1, V_{H} CDR2, and V_{H} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 2, 5, and 8, respectively; or (3) the V_{H} CDR1, V_{H} CDR2, and V_{H} CDR3 comprise amino acid sequences at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 3, 6, and 9, respectively; and/or
ii) a light chain variable region, comprising a V_{L} CDR1, a V_{L} CDR2, and a V_{L} CDR3, wherein (1) the V_{L} CDR1, V_{L} CDR2, and V_{L} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 10, 13, and 16, respectively; (2) the V_{L} CDR1, V_{L} CDR2, and V_{L} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 11, 14, and 17, respectively; or (3) the V_{L} CDR1, V_{L} CDR2, and V_{L} CDR3 comprise amino acid sequences at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 12, 15, and 18, respectively.

In some embodiments, the anti-CD40 antibody or the antigen-binding portion thereof comprises a heavy chain variable region and a light chain variable region; the heavy chain variable region comprises a V_{H} CDR1, a V_{H} CDR2, and a V_{H} CDR3, and the light chain variable region comprises a V_{L} CDR1, a V_{L} CDR2, and a V_{L} CDR3, wherein (1) the V_{H} CDR1, V_{H} CDR2, V_{H} CDR3, V_{L} CDR1, V_{L} CDR2, and V_{L} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 4, 7, 10, 13, and 16, respectively; (2) the V_{H} CDR1, V_{H} CDR2, V_{H} CDR3, V_{L} CDR1, V_{L} CDR2, and V_{L} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 2, 5, 8, 11, 14, and 17, respectively; or (3) the V_{H} CDR1, V_{H} CDR2, V_{H} CDR3, V_{L} CDR1, V_{L} CDR2, and V_{L} CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 3, 6, 9, 12, 15, and 18, respectively.

In some embodiments, the anti-CD40 antibody or the antigen-binding portion thereof comprises a heavy chain variable region comprising an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NOs: 19, 20, 21, or 22, wherein in SEQ ID NO: 20, X1 = A or S.

In some embodiments, the anti-CD40 antibody or the antigen-binding portion thereof comprises a light chain variable region comprising an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NOs: 23, 24, 25, or 26, wherein in SEQ ID NO: 24, X1 = K and X2 = F, or X1 = Y and X2 = Y

In some embodiments, the anti-CD40 antibody or the antigen-binding portion thereof comprises a heavy chain variable region and a light chain variable region, the heavy chain variable region and the light chain variable region comprising: (1) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 19 and 23, respectively; (2) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 20 and 24, respectively, wherein in SEQ ID NO: 20, X1 = A, and in SEQ ID NO: 24, X1 = K and X2 = F; (3) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 20 and 24, respectively, wherein in SEQ ID NO: 20, X1 = S, and in SEQ ID NO: 24, X1 = K and X2 = F; (4) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 20 and 24, respectively, wherein in SEQ ID NO: 20, X1 = A, and in SEQ ID NO: 24, X1 = Y and X2 = Y; (5) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 20 and 24, respectively, wherein in SEQ ID NO: 20, X1 = S, and in SEQ ID NO: 24, X1 = Y and X2 = Y; (6) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 21 and 25, respectively; or (7) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 22 and 26, respectively.

In some embodiments, the anti-CD40 antibody or the antigen-binding portion thereof comprises a heavy chain and a light chain, the heavy chain comprising a heavy chain variable region and a heavy chain constant region, and thelight chain comprising a light chain variable region and a light chain constant region, wherein the C terminus of the heavy chain variable region is linked to the N terminus of the heavy chain constant region, and the C terminus of the light chain variable region is linked to the N terminus of the light chain constant region, wherein the heavy chain variable region and the light chain variable region comprise the amino acid sequences described above; the heavy chain constant region can be a human IgG2 constant region having the amino acid sequence set forth in SEQ ID: NO. 28 or a human IgG1 constant region having the amino acid sequence set forth in SEQ ID NO: 27, and the light chain constant region can be a human κ constant region having the amino acid sequence set forth in SEQ ID NO: 29. The heavy chain constant region, such as Fc fragments, can be engineered to have reduced or enhanced FcR-binding affinity.

In some specific embodiments, the anti-CD40 antibody or the antigen-binding portion thereof comprises a heavy chain and a light chain, wherein: (1) the heavy chain comprises the amino acid sequences set forth in SEQ ID NOs: 19 and 27, and the light chain comprises the amino acid sequences set forth in SEQ ID NOs: 23 and 29; (2) the heavy chain comprises the amino acid sequences set forth in SEQ ID NOs: 20 and 28, and the light chain comprises the amino acid sequences set forth in SEQ ID NOs: 24 and 29, wherein in SEQ ID NO: 20, X1 = A, and in SEQ ID NO: 24, X1 = K and X2 = F; (3) the heavy chain comprises the amino acid sequences set forth in SEQ ID NOs: 20 and 28, and the light chain comprises the amino acid sequences set forth in SEQ ID NOs: 24 and 29, wherein in SEQ ID NO: 20, X1 = S, and in SEQ ID NO: 24, X1 = K and X2 = F; (4) the heavy chain comprises the amino acid sequences set forth in SEQ ID NOs: 20 and 28, and the light chain comprises the amino acid sequences set forth in SEQ ID NOs: 24 and 29, wherein in SEQ ID NO: 20, X1 = A, and in SEQ ID NO: 24, X1 = Y and X2 = Y; (5) the heavy chain comprises the amino acid sequences set forth in SEQ ID NOs: 20 and 28, and the light chain comprises the amino acid sequences set forth in SEQ ID NOs: 24 and 29, wherein in SEQ ID NO: 20, X1 = S, and in SEQ ID NO: 24, X1 = Y and X2 = Y; (6) the heavy chain comprises the amino acid sequences set forth in SEQ ID NOs: 21 and 27, and the light chain comprises the amino acid sequences set forth in SEQ ID NOs: 25 and 29; or (7) the heavy chain comprises the amino acid sequences set forth in SEQ ID NOs: 22 and 27, and the light chain comprises the amino acid sequences set forth in SEQ ID NOs: 26 and 29.

In some embodiments, the anti-CD40 antibody comprises or consists of two heavy chains and two light chains, wherein each of the heavy chains comprises the heavy chain constant region, the heavy chain variable region, or the CDR sequences described above, and each of the light chains comprises the light chain constant region, the light chain variable region, or the CDR sequences described above. The anti-CD40 antibody of the present application can be a full-length antibody, e.g., a full-length antibody of IgG1, IgG2, or IgG4 isotype. In other embodiments, the anti-CD40 antibody or the antigen-binding portion thereof of the present application can be a single chain variable fragment (scFv) antibody or an antibody fragment, such as an Fab or F(ab')₂ fragment. In some embodiments, the anti-CD40 antibody or the antigen-binding portion thereof of the present application binds to CD40 (e.g., human CD40 and monkey CD40). In some embodiments, the anti-CD40 antibody or the antigen-binding portion thereof of the present application blocks the binding of CD40 to CD40L. In some embodiments, the anti-CD40 antibody or the antigen-binding portion thereof of the present application activates the CD40 signal transduction.

Preferably, the anti-CD40 antibody of the present application is a humanized monoclonal antibody. Additionally or alternatively, the anti-CD40 antibody of the present application can be, for example, a chimeric monoclonal antibody.

The amino acid sequence IDs of the heavy/light chain variable regions of exemplary anti-CD40 antibodies or the antigen-binding portions thereof of the present application are summarized in Table 1 below. Some antibodies have the same V_{H} or V_{L}. The heavy chain constant region of the antibody can be a human IgG1 or IgG2 constant region having the amino acid sequences set forth in SEQ ID NOs: 27 and 28, respectively, and the light chain constant region of the antibody can be a human κ constant region having the amino acid sequence set forth in SEQ ID NO: 29. These antibodies can also comprise a mouse IgG1 or IgG2 heavy chain constant region and/or a mouse κ constant region.

**Table 1. Amino acid sequence IDs of heavy/light chain variable regions**

| Antibody | V_{H} CDR1 | V_{H} CDR2 | V_{H} CDR3 | V_{H} | V_{L} CDR1 | V_{L} CDR2 | V_{L} CDR3 | V_{L} |
|---|---|---|---|---|---|---|---|---|
| C1H1 | 1 | 4 | 7 | 19 | 10 | 13 | 16 | 23 |
| huC1H1-V1 | 1 | 4 | 7 | 20, X1=A | 10 | 13 | 16 | 24, X1=K, X2=F |
| huC1H1-V2 | 1 | 4 | 7 | 20, X1=S | 10 | 13 | 16 | 24, X1=K, X2=F |
| huC1H1-V3 | 1 | 4 | 7 | 20, X1=A | 10 | 13 | 16 | 24, X1=Y, X2=Y |
| huC1H1-V4 | 1 | 4 | 7 | 20, X1=S | 10 | 13 | 16 | 24, X1=Y, X2=Y |
| 1A3 | 2 | 5 | 8 | 21 | 11 | 14 | 17 | 25 |
| 1D1 | 3 | 6 | 9 | 22 | 12 | 15 | 18 | 26 |

The CDRs of the heavy chain variable region and the CDRs of the light chain variable region in Table 1 are defined by the Kabat numbering system. However, as is well known in the art, the CDRs can also be determined by other numbering systems such as the Chothia, IMGT, AbM, or Contact numbering system/method based on the sequence of heavy/light chain variable region. It will be understood by those skilled in the art that, unless otherwise specified, the term "CDRs" or "complementarity determining regions" of a given antibody or a region thereof (e.g., a variable region) should be understood to encompass complementarity determining regions defined by any one of the known schemes. Although the protection scope claimed by the present disclosure is based on the sequences set forth in Table 1, amino acid sequences defined according to other CDR numbering systems shall also fall within the protection scope of the present disclosure.

The V_{H} and V_{L} sequences (or CDR sequences) of other anti-CD40 antibodies that bind to human CD40 can be "mixed and paired" with the V_{H} and V_{L} sequences (or CDR sequences) of the anti-CD40 antibody of the present application. Preferably, when V_{H} and V_{L} chains (or CDRs in these chains) are mixed and paired, the V_{H} sequence in a particular V_{H}/V_{L} pair is substituted with a structurally similar V_{H} sequence. Likewise, it is preferred to substitute the V_{L} sequence in a particular V_{H}/V_{L} pair with a structurally similar V_{L} sequence. Therefore, in one embodiment, the antibody or the antigen-binding portion thereof of the present application comprises:
(a) a heavy chain variable region comprising an amino acid sequence listed in Table 1; and
(b) a light chain variable region comprising an amino acid sequence listed in Table 1, or a V_{L} of another anti-CD40 antibody specifically binding to human CD40.

In another embodiment, the antibody or the antigen-binding portion thereof of the present application comprises:
(a) CDR1, CDR2, and CDR3 of heavy chain variable region listed in Table 1; and
(b) CDR1, CDR2, and CDR3 of light chain variable region listed in Table 1, or CDRs of another anti-CD40 antibody specifically binding to human CD40.

In another embodiment, the antibody or the antigen-binding portion thereof of the present application comprises the CDR2 of the heavy chain variable region of the anti-CD40 antibody in the present application and CDRs of other antibodies that bind to human CD40, e.g., CDR1 and/or CDR3 of the heavy chain variable region and/or CDR1, CDR2, and/or CDR3 of the light chain variable region from another anti-CD40 antibody. In addition, it is well known in the art that, independent of the CDR1 and/or CDR2 domains, the CDR3 domain can individually determine the binding specificity of an antibody to a homologous antigen, and that multiple antibodies with the same binding specificity can be predictively generated based on a common CDR3 sequence.

Accordingly, in another embodiment, the antibody of the present application comprises a CDR2 of the heavy chain variable region of the anti-CD40 antibody in the present application and at least a CDR3 of the heavy chain variable region and/or the light chain variable region of the anti-CD40 antibody in the present application, or a CDR3 of a heavy chain variable region and/or light chain variable region of another anti-CD40 antibody, wherein the antibody specifically binds to human CD40. Such antibodies preferably (a) compete with the anti-CD40 antibody of the present application for binding to CD40; (b) retain the functional properties of the anti-CD40 antibody of the present application; (c) bind to the same epitope as the anti-CD40 antibody of the present application; and/or (d) have similar binding affinities to the anti-CD40 antibody of the present application. In another embodiment, the antibody of the present application can further comprise a CDR2 of the light chain variable region of the anti-CD40 antibody in the present application, or a CDR2 of light chain variable region of another anti-CD40 antibody, wherein the antibody specifically binds to human CD40. In another embodiment, the antibody of the present application can further comprise the CDR1 of the heavy chain variable region and/or the light chain variable region of the anti-CD40 antibody in the present application, or the CDR1 of the heavy chain variable region and/or light chain variable region of another anti-CD40 antibody, wherein the antibody specifically binds to human CD40.

In another embodiment, the antibody of the present application comprises a heavy chain variable region and/or a light chain variable region, the heavy chain variable region and light chain variable region each comprising a CDR1, a CDR2, and a CDR3, wherein these CDR sequences are different from the CDR sequences of the anti-CD40 antibody in the present application, and the difference is derived from one or more conservative modifications. It is understood in the art that certain conservative sequence modifications do not eliminate the antigen-binding properties.

Therefore, in one embodiment, the antibody of the present application comprises a heavy chain variable region and/or a light chain variable region, the heavy chain variable region and light chain variable region each comprising CDR1, CDR2, and CDR3 sequences, wherein:
(a) CDR1 sequence of the heavy chain variable region comprises a sequence listed in Table 1, and/or conservative modifications thereof; and/or
(b) CDR2 sequence of the heavy chain variable region comprises a sequence listed in Table 1, and/or conservative modifications thereof; and/or
(c) CDR3 sequence of the heavy chain variable region comprises a sequence listed in Table 1, and/or conservative modifications thereof; and/or
(d) CDR1, CDR2, and/or CDR3 sequences of the light chain variable region comprise sequences listed in Table 1, and/or conservative modifications thereof; and (e) the antibody specifically binds to human CD40.

The term "conservative sequence modification" refers to an amino acid modification that does not significantly affect or alter the binding properties of the antibody. Such conservative modifications include amino acid substitutions, additions, and deletions. Modifications can be introduced into the antibody of the present application using standard techniques known in the art, e.g., point mutation and PCR-mediated mutation. Conservative amino acid substitutions are those in which an amino acid residue is replaced by an amino acid residue having a similar side chain. Amino acid residue groups having similar side chains are known in the art. Such amino acid residue groups include amino acids with basic side chains (e.g., lysine, arginine, and histidine), amino acids with acidic side chains (e.g., aspartic acid and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in the CDRs of the antibody of the present application can be replaced by other amino acid residues from the same set of side chains, and the resultant antibody can be tested for function retention by function assays described in the present application.

In other embodiments, the anti-CD40 antibody or the antigen-binding portion thereof is selected from the group consisting of dacetuzumab, lucatumumab, APX005M, CDX-1140 (Celldex), GEN-1042 (BioNTech; Genmab), selicrelumab, SEA-CD40 (Seagen), 2141-v11 (AbbVie), ChiLob7/4 (BioNTech), mitazalimab, vanalimab, giloralimab, LVGN-7409 (Lyvgen Biopharma), LVGN-7408 (Lyvgen Biopharma), YH003 (Eucure Biopharma), SHR-1704 (Hengrui Pharmaceuticals), MIL97 (Mabworks Biotech), iscalimab, bleselumab, ravagalimab, or an antigen-binding portion of the above antibodies.

### Formulation of Anti-CD40 Antibody or Antigen-Binding Portion Thereof

In some embodiments, a single dose of the formulation of the anti-CD40 antibody or the antigen-binding portion thereof comprises 2 mg to 20 mg, e.g., 2 mg, 4 mg, 6 mg, 8 mg, 10 mg, 12 mg, 14 mg, 16 mg, 18 mg, or 20 mg of the anti-CD40 antibody or the antigen-binding portion thereof.

In some embodiments, the formulation of the anti-CD40 antibody or the antigen-binding portion thereof comprises the anti-CD40 antibody or the antigen-binding portion thereof, and excipients of any amount. Excipients that can be used include carriers, surfactants, thickeners or emulsifiers, solid binders, dispersants or suspensions, solubilizers, coloring agents, flavoring agents, coatings, disintegrants, lubricants, sweeteners, preservatives, isotonic agents, and combinations thereof. The selection and use of suitable excipients are taught in Gennaro, ed., Remington: The Science and Practice of Pharmacy, 20th Ed. (Lippincott Williams & Wilkins 2003), the disclosure of which is incorporated herein by reference.

The formulation of the anti-CD40 antibody or the antigen-binding portion thereof is suitable for intravenous, intramuscular, subcutaneous, parenteral, intraspinal, or intraepidermal administration (e.g., by injection or infusion). Depending on the route of administration, the active ingredient can be encapsulated in materials to protect it from acids and other natural conditions that may inactivate it. The phrase "parenteral administration" used herein refers to modes of administration other than enteral and topical administration that are typically performed by injection, including but not limited to, intravenous, intramuscular, intraarterial, intrathecal, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion. Alternatively, the formulation of the anti-CD40 antibody or the antigen-binding portion thereof can be administered through a non-parenteral route, for example, topical, intraepidermal, or transmucosal route, such as intranasal, oral, vaginal, rectal, sublingual, or topical administration. The administration can also be performed, e.g., once, multiple times, and/or over one or more extended periods of time.

In one specific embodiment, the formulation of the anti-CD40 antibody or the antigen-binding portion thereof is a water-soluble injection, the water-soluble injection including but not limited to unlyophilized water-soluble formulations or water-soluble formulations obtained by reconstitution of lyophilized powders. In other embodiments, the formulation of the anti-CD40 antibody or the antigen-binding portion thereof is a lyophilized formulation. The lyophilized formulation refers to a formulation prepared by subjecting an aqueous solution to a lyophilization process in which the substance is first frozen, and then the amount of the solvent is reduced by sublimation (primary drying process) and then by desorption (secondary drying process) until the amount of the solvent is reduced to a value that no longer supports the biological activity or a chemical reaction. The lyophilized formulation of the present application can also be dried by other methods known in the art, such as spray drying and bubble drying.

### Anlotinib or Pharmaceutically Acceptable Salt Thereof

As used herein, the chemical name of anlotinib (*i.e.,* the compound of formula (I)) is 1-[[[4-(4-fluoro-2-methyl-1H-indol-5-yl)oxy-6-methoxyquinolin-7-yl]oxy]methyl]cyclopropylamine, which has the following structural formula:

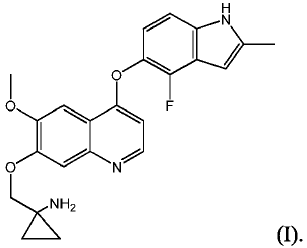

In the present application, anlotinib refers to the compound of formula (I) in any case.

Anlotinib can be administered in its free base form, or in the form of its salt, hydrate, or prodrug thereof which may convert into the free base form of anlotinib *in vivo.* For example, the pharmaceutically acceptable salt of anlotinib is also within the scope of the present application, and can be generated from various organic and inorganic acids according to methods well known in the art.

Further, the pharmaceutically acceptable salt of anlotinib is a salt formed by the compound of formula (I) and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxyl naphthoic acid, salicylic acid and stearic acid; preferably, the pharmaceutically acceptable salt is a hydrochloride or maleate salt, and more preferably a dihydrochloride salt. In some embodiments, it is administered in the form of anlotinib hydrochloride. In some embodiments, it is administered in the form of anlotinib monohydrochloride. In some embodiments, it is administered in the form of anlotinib dihydrochloride. In some embodiments, it is administered in the crystalline form of anlotinib hydrochloride. In one specific embodiment, it is administered in the crystalline form of anlotinib dihydrochloride.

Unless otherwise stated, the dosages of anlotinib or the salt thereof involved in the present application are calculated based on the free base of anlotinib.

### Formulation of Anlotinib or Pharmaceutically Acceptable Salt Thereof

In some embodiments, a single dose of the formulation of anlotinib or the pharmaceutically acceptable salt thereof comprises 6 mg, 8 mg, 10 mg, or 12 mg of anlotinib.

In some embodiments, the formulation of anlotinib or the pharmaceutically acceptable salt thereof includes, but is not limited to, formulations suitable for oral, parenteral, and topical administration. In some embodiments, the formulation of anlotinib or the pharmaceutically acceptable salt thereof is preferably a formulation suitable for oral administration, including tablets, capsules, powders, granules, dripping pills, pastes, pulvis, and the like, preferably tablets and capsules. The tablets can be common tablets, dispersible tablets, effervescent tablets, sustained-release tablets, controlled-release tablets, or enteric-coated tablets. The capsules can be a common capsules, sustained-release capsules, controlled-release capsules, or enteric coated capsules. The oral formulation can be prepared by conventional methods using a pharmaceutically acceptable carrier well known in the art. The pharmaceutically acceptable carrier includes fillers, absorbents, wetting agents, binders, disintegrants, lubricants, and the like. The fillers include starch, lactose, mannitol, microcrystalline cellulose, and the like. The absorbents include calcium sulfate, calcium hydrogen phosphate, calcium carbonate, and the like. The wetting agents include water, ethanol, and the like. The binders include hydroxypropyl methylcellulose, polyvidone, microcrystalline cellulose, and the like. The disintegrants include croscarmellose sodium, crospovidone, surfactants, low-substituted hydroxypropylcellulose, and the like. The lubricants include magnesium stearate, talcum powder, polyethylene glycol, sodium dodecyl sulfate, silica gel micropowder, talcum powder, and the like. The pharmaceutically acceptable carrier further includes coloring agents, sweeteners, and the like.

In one embodiment, the formulation of anlotinib or the pharmaceutically acceptable salt thereof is a solid formulation suitable for oral administration. In one embodiment, the formulation of anlotinib or the pharmaceutically acceptable salt thereof can be in the form of a tablet or a capsule. In one specific embodiment, the formulation of anlotinib or the pharmaceutically acceptable salt thereof is in the form of a capsule. In one specific embodiment, the pharmaceutically acceptable carrier of the oral solid formulation includes mannitol, microcrystalline cellulose, hydroxypropylcellulose, and magnesium stearate.

### Chemotherapeutic Drug

As used herein, the third therapeutic agent can be known therapeutic agents for cancer in the art, such as chemotherapeutic drugs.

In one embodiment, the chemotherapeutic drug is one or more of alkylating agents, podophyllum drugs, platinum-based drugs, fluoropyrimidine derivatives, cytosine derivatives, taxane drugs, camptothecin analog drugs, anthracycline drugs, and vinca alkaloid drugs. The chemotherapeutic drug described herein further includes one or more of pemetrexed, mitomycin, methotrexate, mitoxantrone, bendamustine, temozolomide, sapacitabine, tegafur-gimeracil-oteracil potassium, and encequidar. In one embodiment, the alkylating agent is one or more of cyclophosphamide, ifosfamide, carmustine, and melphalan. In one embodiment, the podophyllum drug is one or more of etoposide, teniposide, and podophyllotoxin glucopyranoside. In one embodiment, the cytosine derivatives are one or more of cytarabine, gemcitabine, and azacitidine. In one embodiment, the platinum-based drug is one or more of oxaliplatin, cisplatin, carboplatin, nedaplatin, and bicycloplatin. In one embodiment, the fluoropyrimidine derivatives is one or more of gemcitabine, capecitabine, fluorouracil, difuradin, doxifluridine, tegafur, carmofur, and trifluridine. In one embodiment, the taxane drug is one or more of paclitaxel, albumin-bound paclitaxel, and docetaxel. In one embodiment, the camptothecin analog is one or more of camptothecin, hydroxycamptothecin, irinotecan, and topotecan. In one embodiment, the anthracycline is one or more of adriamycin, epirubicin, daunorubicin, pirarubicin, amrubicin, and aclacinomycin. In one embodiment, the vinca alkaloid is one or more of vinorelbine, vinblastine, vincristine, vindesine, and vinflunine.

In some embodiments, the chemotherapeutic drug is administered according to known modes of administration (including doses, routes of administration, and regimens).

### Cancer

The term "cancer" described herein refers to any malignant and/or invasive growth or tumor caused by abnormal cell growth, including solid tumors, blood cancers, bone marrow cancers, or lymphatic system cancers named after the cell types from which they form. The term "cancer" includes, but is not limited to, a primary cancer originating at a specific site in the body, a metastatic carcinoma that has spread from its initial location to other sites in the body, the recurrence of a primary cancer after remission, and a second primary cancer (a new primary cancer in a patient with a history of a cancer of different type).

In certain aspects, the cancers described herein are solid tumors. In some embodiments, the solid tumors includes, but are not limited to: skin cancer such as melanoma and sarcoma, pancreatic cancer, colorectal cancer such as colorectal adenocarcinoma, esophageal cancer, gastrointestinal cancer, prostate cancer, bladder cancer, kidney cancer, ovarian cancer, uterine cancer such as cervical cancer and endometrial cancer, breast cancer such as triple-negative breast cancer, lung cancer such as small cell lung cancer and non-small cell lung cancer, thyroid cancer, nasopharyngeal carcinoma, and liver cancer.

In certain aspects, the cancer described herein is a B cell malignancy. The term "B cell malignancy" includes any malignancy derived from cells of the B cell lineage. In some embodiments, the B cell malignancy includes leukemia and lymphoma, including but not limited to, non-Hodgkin's lymphoma, chronic lymphocytic leukemia, multiple myeloma, B cell lymphoma, high-grade B cell lymphoma, medium-grade B cell lymphoma, low-grade B cell lymphoma, B cell acute lymphocytic leukemia, Hodgkin's lymphoma, plasmacytoma, follicular lymphoma, follicular small cleaved cell lymphoma, follicular large cell lymphoma, follicular mixed small cleaved cell lymphoma, diffuse small cleaved cell lymphoma, small lymphocytic lymphoma, prolymphocytic leukemia, lymphoplasmacytic lymphoma, marginal zone lymphoma, mucosa-associated lymphoid tissue lymphoma, monocytic B cell lymphoma, splenic leukemia, hairy cell leukemia, diffuse large B cell lymphoma, mediastinal large B cell lymphoma, lymphomatoid granulomatosis, intravascular lymphoma, mixed lymphoma, immunoblastic lymphoma, Burkitt lymphoma, AIDS-related lymphoma, Waldenstrom's macroglobulinemia, and mantle cell lymphoma.

In certain aspects, the cancer described herein is a non-B cell hematological malignancy. In some embodiments, the non-B cell hematological malignancy includes, but is not limited to, chronic myelogenous leukemia and acute myelogenous leukemia.

### Administration

The content below is not intended to limit the administration of the pharmaceutical combination disclosed herein.

The components in the pharmaceutical combination of the present application can be administered independently, or some or all of the components are administered in any suitable route, including, but not limited to, oral administration or parenteral administration (by intravenous, intramuscular, topical, or subcutaneous routes). In some embodiments, the components in the pharmaceutical combination of the present application may be administered independently, or some or all of the components can be administered by means of oral administration or injection, for example, intramuscular injection, intravenous injection, subcutaneous injection, intratumoral injection, or intraperitoneal injection.

The components of the pharmaceutical combination of the present application can be independently formulated into suitable dosage forms, or some or all of the components can be formulated into a suitable dosage form including, but not limited to, tablets, lozenges, pills, capsules (for example, hard capsules, soft capsules, enteric capsules, or microcapsules), elixirs, granules, syrups, injections (for example, intramuscular, intravenous, subcutaneous, intratumoral, or intraperitoneal), granules, emulsions, suspensions, solutions, dispersant, and dosage forms of sustained-released preparations for oral or non-oral administration.

The components in the pharmaceutical combination disclosed herein can be formulated independently, or some or all of the components are formulated with a pharmaceutically acceptable carrier and/or excipient.

### Technical Effects

Generally, the use of the pharmaceutical combination of the present application described above will provide:
(1) better efficacy in controlling tumor growth or even eliminating tumors as compared with any drug of the pharmaceutical combination administered alone;
(2) fewer doses as compared with any drug of the pharmaceutical combination administered alone;
(3) good tolerability in patients and fewer adverse effects and/or complications as compared with any drug of the pharmaceutical combination administered alone;
(4) a higher disease control rate in patients treated;
(5) a prolonged survival (e.g., median survival, progression-free survival, or overall survival) in patients treated;
(6) a prolonged survival (e.g., median survival, progression-free survival, or overall survival) in patients treated as compared with standard chemotherapies;
(7) a prolonged duration of response (DOR); and/or
(8) better anti-cancer activity and a better anti-cancer synergistic effect as compared with any drug of the pharmaceutical combination administered alone.

### Definitions and Explanations

For a better understanding of the present disclosure, terms are defined herein. Other definitions are listed throughout the detailed description. Unless otherwise stated, the following terms used in the present application shall have the following meanings. A certain term, unless otherwise specifically defined, should not be considered ambiguous or unclear, but construed according to its common meaning in the art. When referring to a trade name, it is intended to refer to its corresponding commercial product or its active ingredient.

The term "CD40" refers to the tumor necrosis factor receptor superfamily member 5 (TNFR5). The term "CD40" includes variants, isoforms, homologs, orthologs, and paralogs. For example, in certain instances, an antibody specific for human CD40 protein can cross-react with CD40 protein from a species other than a human (e.g., monkey). In other embodiments, an antibody specific for human CD40 protein can be completely specific for human CD40 protein and does not cross-react with proteins of other species or other types, or can cross-react with CD40 derived from some other species but not all other species.

The term "human CD40" refers to a CD40 protein having a human amino acid sequence, such as the human CD40 amino acid sequence with Genbank accession No. NP_001241.1 (Amini M et al., (2020) Life Sci 254:117774). The terms "monkey or rhesus CD40" and "mouse CD40" refer to the monkey CD40 sequence and the mouse CD40 sequence, respectively, e.g., those having the amino acid sequences with Genbank accession Nos. NP_001252791.1 and NP_035741.2.

The term "immune response" refers to the action of, for example, lymphocytes, antigen-presenting cells, phagocytes, granulocytes, and soluble macromolecules produced by the above cells or liver (including antibodies, cytokines, and complements) in a human body to selectively damage, destroy, or eliminate invading pathogens, pathogen-infected cells or tissues, cancer cells, or normal human cells or tissues in the case of autoimmunity or pathological inflammation.

The term "antibody" refers to an immunoglobulin molecule that recognizes and specifically binds to a target (e.g., CD40) through at least one antigen-binding site, which is typically located within the variable region of the immunoglobulin molecule. As used herein, the term "antibody" encompasses intact polyclonal antibodies, intact monoclonal antibodies, single-chain Fv (scFv) antibodies, heavy chain antibodies (HCAbs), light chain antibodies (LCAbs), multispecific antibodies, bispecific antibodies, monospecific antibodies, a monovalent antibodies, fusion proteins comprising an antigen-binding site of an antibody, and any other modified immunoglobulin molecules (e.g., a dual-variable-domain immunoglobulin molecule) comprising an antigen-binding site, so long as the antibody exhibits the desired biological activity. The antibody further includes, but is not limited to, a mouse antibody, a chimeric antibody, a humanized antibody, and a human antibody. The antibody can be any of the five major classes of immunoglobulins: IgA, IgD, IgE, IgG and IgM depending on the characteristics of the heavy chain constant region of an antibody (referred to as α, δ, ε, γ, and µ, respectively), or subclasses (isotypes) thereof (e.g., IgG1, IgG2, IgG3, IgG4, IgA1, and IgA2). Different classes of immunoglobulins have different subunit structures and three-dimensional configurations that are well known in the art. The antibody may be a naked antibody or a conjugate with other molecules, including but not limited to toxins and radioisotopes. Unless otherwise specified, the term "antibody" used herein includes the "antigen-binding portion" of an intact antibody. IgG is a glycoprotein comprising two heavy (H) chains and two light (L) chains, wherein the heavy chains and the light chains are linked by disulfide bonds. Each of the heavy chains is composed of a heavy chain variable region (abbreviated as V_{H}) and a heavy chain constant region. The heavy chain constant region is composed of three domains: C_{H1}, C_{H2}, and C_{H3}. Each of the light chains is composed of a light chain variable region (abbreviated as V_{L}) and a light chain constant region.

The light chain constant region is composed of one domain C_{L}. The V_{H} and V_{L} regions may also be further divided into hypervariable regions, known as complementarity determining regions (CDRs), which are separated by more conservative framework regions (FRs). V_{H} and V_{L} are each composed of three CDRs and four FRs, arranged from the amino-terminus to the carboxyl-terminus in the following order: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component (C1q) of the classical complement system.

The "antigen-binding portion" of an antibody (or abbreviated as "antibody portion") refers to one or more fragments of the antibody that retain the ability to specifically bind to an antigen (e.g., CD40 protein). It has been demonstrated that the antigen-binding functionality of an antibody can be implemented by fragments of the full-length antibody. Examples of binding fragments encompassed within the "antigen-binding portion" of an antibody include: (i) an Fab fragment, a monovalent fragment composed of V_{L}, V_{H}, C_{L} and C_{H1} domains; (ii) an F(ab')₂ fragment, a bivalent fragment comprising two Fab fragments linked by a disulfide bond at the hinge region; (iii) an Fd fragment composed of V_{H} and C_{H1} domains; (iv) an Fv fragment composed of V_{L} and V_{H} domains of a single arm of the antibody; (v) a dAb fragment composed of a V_{H} domain (Ward et al., (1989) Nature 341:544-546); (vi) isolated complementarity determining regions (CDRs); and (vii) a nanobody, a heavy chain variable region comprising a single variable domain and two constant domains. Furthermore, although the two domains V_{L} and V_{H} of the Fv fragment are encoded by different genes, the V_{L} and V_{H} can be joined via a synthetic linker by recombinant means into a single protein chain in which V_{L} and V_{H} pair to form a monovalent molecule (referred to as single-chain Fv (scFv); see, e.g., Bird et al., (1988) Science 242:423-426; and Huston et al., (1988) Proc. Natl. Acad. Sci. USA 85:5879-5883). Such single-chain antibodies are also encompassed within the term "antigen-binding portion" of an antibody. These antibody fragments can be obtained using conventional techniques known to those skilled in the art, and the fragments can be subjected to functional screening using the same method as intact antibodies.

The term "isolated antibody" refers to an antibody that is substantially free of other antibodies with different antigenic specificities (e.g., an isolated antibody that specifically binds to CD40 protein is substantially free of antibodies that specifically bind to antigens other than CD40 protein). However, an isolated antibody that specifically binds to human CD40 protein can be cross-reactive to other antigens, such as CD40 proteins of other species. Furthermore, the isolated antibody is substantially free of other cellular materials and/or chemical substances.

The term "mouse antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The mouse antibody of the present application can comprise amino acid residues not encoded by mouse germline immunoglobulin sequences (e.g., mutations introduced by *in-vitro* random or point mutations, or by *in-vivo* somatic mutations). However, the term "mouse antibody" as used herein does not include antibodies in which the CDR sequences from other mammalian species are inserted between the mouse framework region sequences.

The term "chimeric antibody" refers to an antibody obtained by combining genetic substances of non-human origin with genetic substances of human origin. More generally, a chimeric antibody refers to an antibody that combines genetic substances of one species with genetic substances of another species.

The term "humanized antibody" refers to an antibody from a non-human species of which the protein sequence has been modified to increase its similarity to a natural human antibody.

The "antigen-recognizing antibody" and "antibody specific for an antigen/antibody having specificity for an antigen" are used herein interchangeably with the term "antibody specifically binding to an antigen".

An antibody that "specifically binds to human CD40" or the antigen-binding portion thereof refers to an antibody that binds to human CD40 protein (or possibly further including CD40 proteins from one or more non-human species) but does not substantially bind to a non-CD40 protein. Preferably, the antibody binds to human CD40 protein with "high affinity", i.e., binding to human CD40 protein with a K_{D} value of 5.0 × 10⁻⁸ M or less, preferably 1.0 × 10⁻⁸ M or less.

The term "substantially do not bind to" a protein or cell refers to the absence of binding to the protein or cell, or the absence of binding to the protein or cell with high affinity, i.e., binding to the protein or cell with a K_{D} value of 1.0 × 10⁻⁶ M or greater, preferably 1.0 × 10⁻⁵ M or greater, more preferably 1.0 × 10⁻⁴ M or greater, even more preferably 1.0 × 10⁻³ M or greater, and still more preferably 1.0 × 10⁻² M or greater.

For an IgG antibody, the term "high affinity" refers to that the antibody has a K_{D} value for the target antigen of 1.0 × 10⁻⁶ M or less, preferably 9.0 × 10⁻⁹ M or less, more preferably 5.0 × 10⁻⁹ M or less, even more preferably 1.0 × 10⁻⁹ M or less, and still more preferably 5.0 × 10⁻¹⁰ M or less. However, for other antibody isotypes, the "high affinity" binding may be different. For example, for IgM isotype, the "high affinity" refers to that an antibody has a K_{D} value of 10⁻⁶ M or less, preferably 10⁻⁷ M or less, and more preferably 10⁻⁸ M or less.

The term "K_{assoc}" or "Kₐ" refers to the association rate of a particular antibody-antigen interaction, and the term "K_{dis}" or "K_{d}" used herein refers to the dissociation rate of a particular antibody-antigen interaction. As used herein, the term "K_{D}" refers to the dissociation constant, which is the ratio of to Kₐ (i.e., K_{d}/Kₐ) and is expressed in molar concentration (M). The K_{D} value of an antibody can be determined using methods well known in the art. A preferred method for determining the K_{D} value of an antibody is surface plasmon resonance technology, preferably using a biosensor system, such as the Biacore^{™} system.

The term "EC₅₀", also known as half-maximal effective concentration, refers to the concentration of an antibody that induces a response halfway between the baseline and the maximum after a particular exposure time. The term "IC₅₀", also known as half-maximal inhibitory concentration, refers to the concentration of an antibody that inhibits a specific biological or biochemical function by 50% relative to the case where the antibody is absent.

The term "agonistic CD40 antibody" or "agonistic anti-CD40 antibody" refers to an anti-CD40 antibody that binds to CD40 and activates or induces the CD40 signal transduction (thereby, for example, promoting the activation and proliferation of immune cells and the production of cytokines and chemokines). The term "antagonistic CD40 antibody/antagonistic anti-CD40 antibody" refers to an anti-CD40 antibody that blocks or inhibits the CD40 signal transduction that can be involved in induction by CD40L. The agonistic CD40 antibody promotes innate and adaptive immune responses to tumors in subjects by enhancing the antigen presenting ability of APCs, activating tumor-specific CD4⁺ and CD8⁺ T cells, stimulating the secretion of cytokines and chemokines by lymphocytes and monocytes, enhancing the killing effects on tumor cells by cytotoxic lymphocytes and NK cells, and the like.

As used herein, a percent "identity" in the context of two or more nucleic acids or polypeptides refers to that two or more sequences or subsequences have identical nucleotides or amino acid residues or a specified percentage of identical nucleotides or amino acid residues with or without conservative amino acid substitutions as part of the sequence identity, when compared and aligned for the maximum correspondence (i.e., gaps can be introduced if necessary). The percent identity can be measured using sequence alignment software or algorithms or by visual inspection. Various software and algorithms for amino acid or nucleotide sequence alignment are well known in the art, including but not limited to BLAST, ALIGN, Megalign, BestFit, GCG Wisconsin Package, and variants thereof. In some embodiments, when two nucleic acids or polypeptides described herein are substantially identical, it means that, when compared and aligned for the maximum correspondence, the two nucleic acids or polypeptides have at least 70%, at least 75%, at least 80%, at least 85%, or at least 90%, and in some embodiments, at least 95%, 96%, 97%, 98%, or 99% nucleotide or amino acid residue identity, as measured by a sequence alignment algorithm or visual inspection.

As used herein, the term "pharmaceutical combination" refers to a combination of two or more active ingredients (administered as the respective active ingredients themselves, or as their respective derivatives like pharmaceutically acceptable salts or esters, prodrugs, or compositions) that are administered simultaneously or sequentially. As used herein, the terms "therapeutic combination" and "pharmaceutical combination" are used interchangeably. The active ingredients can be administered to a subject simultaneously or sequentially in any order as individual formulations.

The term "treatment" usually refers to operations for acquiring a desired pharmacological effect and/or physiological effect. In terms of fully or partially preventing a disease or a symptom thereof, the effect can be preventive; and/or in terms of partially or fully stabilizing or curing the disease and/or a side effect of the disease, the effect can be therapeutic. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application for (i) treating a specific disease, condition, or disorder; (ii) alleviating, ameliorating, or eliminating one or more symptoms of a specific disease, condition, or disorder, or (iii) preventing or delaying the onset of one or more symptoms of a specific disease, condition, or disorder described herein. In the case of cancer, an effective amount of a drug can reduce the number of cancer cells, reduce the tumor volume, inhibit (i.e., slow to some extent, preferably stop) the infiltration of cancer cells into peripheral organs, inhibit (i.e., slow to some extent, preferably stop) tumor metastasis, inhibit tumor growth to some extent, and/or reduce to some extent one or more symptoms associated with the cancer. For the extent of inhibiting the growth of cancer cells and/or killing existing cancer cells, it may be cytostatic and/or cytotoxic. The amount of the active substance (e.g., the compound of the present application) constituting the "effective amount" may vary according to factors such as the disease state, age, sex, and weight of the individual, and the ability of the therapeutic agent or therapeutic agent combination to elicit a desired response in the individual. The effective amount may also be determined routinely by those skilled in the art in accordance with their knowledge and the present disclosure.

The terms "administer", "administration", and "administering" refer to physically introducing the composition comprising a therapeutic agent to a subject using any of a variety of methods and delivery systems known to those skilled in the art.

The term "flat dose" refers to a dose administered to a patient without considering the weight or the body surface area (BSA) of the patient. Thus, the flat dose is specified as the absolute amount of a medicament (e.g., anti-CD40 antibody) rather than the mg/kg dose. For example, a 60-kg human and a 100-kg human will receive the same dose of antibody.

The term "fixed combination" refers to the administration of the active components (for example, the anti-CD40 antibody or the compound of formula (I)) to a subject simultaneously at a fixed total dose or in a fixed dose ratio, or in the form of a single entity, pharmaceutical composition, or formulation.

The term "non-fixed combination" refers to the simultaneous, parallel or sequential and temporally unlimited administration of two or more active components as independent entities (for example, pharmaceutical compositions and formulations) to a subject, wherein the active ingredients administered to the subject reach therapeutically effective amounts. An enumerable example of the non-fixed combination is cocktail therapy, for example, 3 or more active components are administered. In a non-fixed combination, each of the active components can be packaged, sold, or administered as a fully independent formulation. The term "non-fixed combination" also includes the combined use of "fixed combinations", or a "fixed combination" and an independent entity of any one or more active components.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, acetate, trifluoroacetate, fumarate, maleate, citrate, succinate, mesylate, benzenesulfonate, or p-methylbenzenesulfonate, preferably, hydrochloride, hydrobromide, sulfate, acetate, trifluoroacetate, fumarate, maleate, mesylate, p-methylbenzenesulfonate, sodium salt, potassium salt, ammonium salt, amino acid salt, etc.

As used herein, the terms "subject" and "patient" are used interchangeably. In some embodiments, the term "subject" or "patient" refers to a mammal. In some embodiments, the subject or patient is a mouse. In some embodiments, the subject or patient is a human.

As used herein, "combined use" or "use in combination" means that two or more active substances may be administered to a subject simultaneously or sequentially in any order as individual formulations.

The term "single dose" refers to the smallest unit of packaging containing a certain quantity of pharmaceutical product; for example, in a box of seven capsules, each capsule is a single dose; or a vial of injection is a single dose. The term "multiple doses" consists of multiple single doses.

The term "formulation" refers to a mixture consisting of one or more of the active ingredients or pharmaceutical combinations thereof of the present application and a pharmaceutically acceptable excipient. The formulation is intended to facilitate the administration of the compound or the pharmaceutical combination thereof in the present application to a subject. As used herein, the terms "pharmaceutical composition" and "formulation" have the same meaning and are used interchangeably.

As used herein, unless otherwise indicated, any concentration range, percentage range, ratio range, or integer range shall be interpreted as including the value of any integer within the listed range and including, when appropriate, fractions thereof (such as one tenth and one hundredth of the integer).

As used herein, "about" or "approximately" means being within an acceptable error range determined by those of ordinary skills in the art for a particular value, which will depend in part on how the value is measured or determined, i.e., the limitation by the measurement system. For example, "about" or "approximately" may mean being within 1 or more than 1 standard deviation, as practiced in the art. Alternatively, "about" or "approximately" may mean a range of up to ± 5%, for example, fluctuating within a particular numerical range given ± 2%, ± 1%, or ± 0.5%. Where a particular value is given in the present application or in the claims, unless otherwise stated, "about" or "approximately" should be interpreted as being within an acceptable error range for that particular value. In this context, unless otherwise indicated, the values of the parameters or conditions in a step are all modified by "about" by default.

### BRIEF DESCRIPTION OF THE DRAWINGS

FIGs. 1A-1C show the binding ability of chimeric antibodies 1A3 (A), 1D1 (B), and C1H1 (C) to human CD40 in capture ELISA.
FIGs. 2A-2C show the activity of chimeric antibodies 1A3 (A), 1D1 (B), and C1H1 (C) to activate CD40 signal transduction in cell-based reporter assay.
FIG. 3 shows the binding ability of humanized antibodies huC1H1-V1 and huC1H1-V2 to human CD40 in capture ELISA.
FIG. 4 shows the binding ability of humanized antibodies huC1H1-V1 and huC1H1-V2 to 293T cells expressing human CD40 in cell-based binding FACS.
FIG. 5 shows the ability of humanized antibodies huC1H1-V1 and huCIHI-V2 to block the binding of human CD40 to CD40L in competitive ELISA.
FIG. 6 shows the ability of humanized antibodies huC1H1-V1 and huC1H1-V2 to block the binding of the benchmark to human CD40 in competitive ELISA.
FIG. 7 shows the activity of humanized antibodies huC1H1-V1 and huC1H1-V2 to activate CD40 signal transduction in cell-based reporter assay.
FIGs. 8A-8B show the individual tumor volume (A) and individual tumor weight (B) in all groups of mice at the end of study (D14), wherein **P < 0.01 and ***P < 0.001 were determined as compared with the IgG2 group by the Mann-Whitney test; **P < 0.01 and ***P < 0.001 were based on the comparison with the huC1H1-V2 + anlotinib dihydrochloride group by the two-tailed Student's t test; P < 0.05 was defined as a statistically significant difference.

### Examples

For clarity, the present application is further described with the following examples, which are, however, not intended to limit the scope of the present application.

The preparation and purification methods of the anti-CD40 antibody or the antigen-binding portion thereof in the present application are described in Patent Publication No. PCT/CN2021084013, which is incorporated herein by reference in its entirety.

The mouse anti-CD40 antibody or the mouse anti-CD40 monoclonal antibody in the examples was produced by hybridoma technique and *in vitro* functional screening according to the method described in Patent No. PCT/CN2021084013. The anti-CD40 antibody huC1H1-V2 in the present application is the anti-CD40 antibody huC1H1-V2 in PCT/CN2021084013.

### Example 1 Affinity determination of anti-CD40 antibody by BIACORE surface plasmon resonance

The affinity and binding kinetics of the anti-CD40 antibody in the present application were characterized by a Biacore T200 system (GE Healthcare, Pittsburgh, PA, USA).

Briefly, a goat anti-human IgG (GE Healthcare, Cat. No. BR100839, human antibody capture kit) was covalently linked to a CM5 chip (GE Healthcare, Cat. No. BR100530, carboxymethylated dextran-coated chip) via the primary amine group using a standard amine coupling kit supplied by Biacore (GE Healthcare, Pittsburgh, PA, USA). Unreacted portions on the biosensor surface were blocked with ethanolamine. Then, the anti-CD40 antibody of the present application and two benchmark antibodies (BM1 (dacetuzumab, Genentech Inc., also referred to CD40-BM1, prepared in-house; the heavy and light chain amino acid sequences are set forth in SEQ ID NOs: 40 and 41, respectively) and BM2 (selicrelumab, Abgenix Inc., also referred to CD40-BM2, prepared in-house; the heavy and light chain amino acid sequences are set forth in SEQ ID NOs: 42 and 43, respectively)) at a concentration of 66.67 nM were directed through the chip at a flow rate of 10 µL/min. Then, serially diluted recombinant human CD40-his (Aero biosystems, Cat. No. CD0-H5228, serially 2-fold diluted from 80 nM in HBS-EP⁺ buffer (supplied by Biacore)) or cynomolgus monkey CD40-his protein (Aero biosystems, Cat. No. CD0-C52H6, serially 2-fold diluted from 80 nM in HBS-EP⁺ buffer (supplied by Biacore)) was directed through the chip at a flow rate of 30 µL/min. The antigen-antibody binding kinetics was tracked for 2 min and the dissociation kinetics was tracked for 10 min. The association and dissociation curves were fitted to a 1:1 Langmuir binding model using BIAcore evaluation software. K_{D}, Kₐ, and K_{d} values were determined.

### Example 2 Binding activity of anti-CD40 antibody

The binding activity of the anti-CD40 antibody was determined by capture ELISA and flow cytometry (FACS).

For capture ELISA, an affinity-purified goat anti-human IgG (F(ab')₂ fragment-specific, Jackson Immuno Research, Cat. No. 109-005-097) in PBS at a concentration of 2 µg/mL was immobilized on a 96-well plate at 100 µL/well and incubated overnight at 4 °C. The plate was washed once with a washing buffer (PBS + 0.05% v/v of Tween-20, PBST) and then blocked with 200 µL/well blocking buffer (PBST containing 5% w/v of skim milk) for 2 h at 37 °C. The plate was washed 4 times, and the anti-CD40 antibody of the present application, BM1, BM2, and a negative control hIgG (human immunoglobulin for intravenous injection (pH 4), Hualan Biological Engineering Inc.) serially 5-fold diluted from 66.7 nM in a PBST containing 2.5% w/v of skim milk was added at 100 µL per well. The plate was incubated for 40 min at 37 °C and washed 4 times. A biotinylated human CD40-Fc protein (amino acid residues 21-193 of SEQ ID NO: 30 linked to the N terminus of amino acid residues 99-330 of SEQ ID NO: 28, dissolved in PBST containing 2.5% w/v of skim milk at a concentration of 1.15 nM) was added at 100 µL/well to a 96-well plate coated with the anti-CD40 antibody. The plate was incubated at 37 °C for 40 min and washed 4 times. An HRP-labeled streptavidin (diluted at 1:10000 in PBST, Jackson Immuno Research, Cat. No. 016-030-084) was added at 100 µL/well, and the plate was incubated at 37 °C for another 40 min. After the last wash, 100 µL/well of ELISA substrate TMB (Innoreagents, Cat. No. TMB-S-002) was added and the plate was incubated at room temperature. After 3-10 min, the reaction was stopped by adding 1 M H₂SO₄ at 50 µL/well, and the absorbance of each well was read on a microplate reader using the dual wavelength mode (TMB detection wavelength: 450 nm, reference wavelength: 630 nm). Curves of OD(450-630) values vs. antibody concentration were plotted. Data were analyzed using Graphpad Prism software, and EC₅₀ values were obtained.

The binding of the anti-CD40 antibody to 293T-CD40 cells (prepared by Biosion) that stably express full-length human CD40 (uniprot # P25942-1, SEQ ID NO: 30) on the cell membrane was measured by flow cytometry (FACS). According to the instructions of lipofectamine 3000 transfection reagent (Thermo Fisher), 293T-CD40 cells were prepared by transfecting 293T cells with pCMV-T-P plasmid inserted with CD40 coding sequence between *EcoRI* and *XbaI* loci. Specifically, 293T-CD40 cells were harvested from cell culture flasks, washed twice, and resuspended in FACS buffer (phosphate buffered saline (PBS) containing 2% v/v of fetal bovine serum). The anti-CD40 antibody of the present application or the benchmark serially 4-fold diluted in the FACS buffer from 80 nM was added at 100 µL/well to a 96-well plate containing 2 × 10⁵ cells/well. The plate was incubated in an ice bath for 40 min. After the cells were washed twice with the FACS buffer, an affinity-purified R-phycoerythrin-labeled goat anti-human IgG (Fcy fragment-specific, Jackson Immuno Research, Cat. No. 109-115-098, 1:1000 diluted in the FACS buffer) was added at 100 µL per well. After incubation at 4 °C for 40 min in the dark, the cells were washed thrice and resuspended in the FACS buffer. Fluorescence values were measured using Becton Dickinson FACS Canto II-HTS, and curves of fluorescence value vs. antibody concentration were plotted. Data were analyzed using Graphpad Prism software, and EC₅₀ values were obtained.

### Example 3 Blocking activity of anti-CD40 antibody against CD40-CD40L binding or CD40-Benchmark binding

### 3.1 Ligand blocking ELISA

The ability of the anti-CD40 antibody to block the binding of CD40 to CD40L was determined by competitive ELISA. Briefly, a human CD40-Fc protein (amino acid residues 21-193 of SEQ ID NO: 30 linked to the N terminus of amino acid residues 99-330 of SEQ ID NO: 28) dissolved in the immobilization buffer (carbonate/bicarbonate buffer) at a concentration of 2 µg/mL was immobilized on a 96-well plate at 100 µL/well and incubated overnight at 4 °C. The next day, the plate was washed once with a washing buffer (PBS + 0.05% v/v of Tween-20, PBST) before a PBST containing 5% w/v of skim milk was added. The plate was then incubated at 37 °C for 2 h for blocking. The plate was then washed 4 times with the washing buffer.

The anti-CD40 antibody of the present application or the benchmark was serially 5-fold diluted from 200 nM in a PBST containing 2.5% w/v of skim milk, and the serially diluted anti-CD40 antibody or benchmark was added at 100 µL/well to a plate coated with CD40-Fc protein and incubated at 37 °C for 40 min. The plate was then washed 4 times with the washing buffer before a biotinylated human CD40L-his protein (Sino Biological Inc., Cat. No. 10239-H08E, 95 ng/mL) was added at 100 µL per well. The plate was then incubated for 40 min at 37 °C. The plate was washed again with the washing buffer. Then, an HRP-labeled streptavidin (Jackson Immunoresearch, Cat. No. 016-030-084, 1:10000 diluted in PBST buffer) was added at 100 µL/well and the plate was incubated at 37 °C for 40 min. The plate was washed again with the washing buffer. Finally, TMB was added and the reaction was stopped by adding 1 M H₂SO₄. The absorbance of each well was read on a microplate reader using the dual wavelength mode (TMB detection wavelength: 450 nm, reference wavelength: 630 nm). Curves of OD(450-630) values vs. antibody concentration were plotted. Data were analyzed using Graphpad Prism software, and IC₅₀ values were obtained.

### 3.2 Benchmark blocking ELISA

The ability of the anti-CD40 antibody of the present application to block the binding of the benchmark to human CD40 was determined by competitive ELISA. Briefly, BM2 antibody was immobilized on a 96-well micro plates at 1 µg/mL in PBS, 100 µL/well, and incubated overnight at 4 °C. The next day, the plate was washed once with a washing buffer before a blocking buffer (PBST containing 5% w/v of skim milk) was added. The plate was then incubated at 37 °C for 2 h for blocking. During the blocking of the 96-well plate, the anti-CD40 antibody of the present application or the benchmark was serially 5-fold diluted from 66.7 nM with a biotinylated human CD40-Fc protein (amino acid residues 21-193 of SEQ ID NO: 30 linked to the N terminus of amino acid residues 99-330 of SEQ ID NO: 28, dissolved in PBST containing 2.5% w/v of skim milk at a concentration of 0.23 nM) and then incubated at room temperature for 40 min. After the plate was washed 4 times, the antibody/biotinylated human CD40-Fc mixture was added at 100 µL/well to the plate coated with BM2. The plate was incubated at 37 °C for 40 min and again washed 4 times with the washing buffer. An HRP-labeled streptavidin was added at 100 µL/well, and the plate was incubated at 37 °C for 40 min to detect biotinylated human CD40-Fc immobilized on the plate. Again, the plate was washed 4 times with the washing buffer. Finally, TMB was added and the reaction was stopped by adding 1 M H₂SO₄. The absorbance was read on a microplate reader using the dual wavelength mode (TMB detection wavelength: 450 nm, reference wavelength: 630 nm). Curves of OD(450-630) values vs. antibody concentration were plotted. Data were analyzed using Graphpad Prism software, and IC₅₀ values were obtained.

### Example 4 Cell-based reporter assay of anti-CD40 antibody

The agonistic activity of the anti-CD40 antibody of the present application was further determined in a CD40 expression reporter cell line 293T-NF-xB-Luc-CD40, which stably expresses full-length human CD40 (uniprot No. P25942-1, SEQ ID NO: 30). According to the instructions of lipofectamine 3000 transfection reagent (Thermo Fisher), a pGL4.32[luc2P NF-xB-RE Hygro] vector (Promega, GenBank^{®} Accession No. EU581860) and a pCMV-T-P plasmid (with a CD40 coding sequence inserted between the *EcoRI* and *XbaI* loci) were transfected into 293T cells in sequence to prepare 293T-NF-κB-Luc-CD40 cells. Upon the contact of the CD40 agonist with the cells, CD40 signaling is activated and luciferase expression is up-regulated which can be detected by luminescence measurement.

Briefly, 293T-NF-κB-Luc-CD40 cells at the log phase were resuspended in DMEM medium (Gibco Inc., Cat. No. 10566-016) containing 10% of FBS (Gibco Inc., Cat. No. 10099-141), and seeded on a 384-well plate (Corning, Cat. No. 3707) at 20 µL/well (containing 5 × 10³ cells). Then, the anti-CD40 antibody of the present application or the controls (including an in-house prepared anti-CD22 antibody as a negative control) serially 3-fold diluted from 200 nM in the medium was added at 20 µL per well and incubated at 37 °C for 6 h. Then, the reagents of ONE-Glo^{™} luciferase assay system (Promega, Cat. No. E6120) were added at 30 µL per well and the plate was incubated for 5 min at room temperature. The chemiluminescence values were measured using Tecan Infinit^{®} 200 Pro. Data were analyzed using Graphpad Prism software, and EC₅₀ values were obtained.

### Example 5 Production and characterization of chimeric antibodies

The heavy chain variable region and the light chain variable region of the mouse anti-CD40 monoclonal antibody were sequenced and summarized in Table 1.

The heavy chain variable region and the light chain variable region of the mouse anti-CD40 monoclonal antibodies C1H1, 1D1, or 1A3 were cloned into a fragment of the human IgG1 heavy chain constant region (SEQ ID NO: 27) and a fragment of the human κ light chain constant region (SEQ ID NO: 29), respectively, wherein the C termini of the variable regions were linked to the N termini of the respective constant regions. The vector containing the nucleotides encoding the heavy chain variable region linked to the human IgG1 heavy chain constant region and the vector containing the nucleotides encoding the light chain variable region linked to the human κ light chain constant region were transiently transfected into 50 mL of 293F cell suspension with 1 mg/mL PEI in a light chain construct-to-heavy chain construct ratio of 1.1:1.

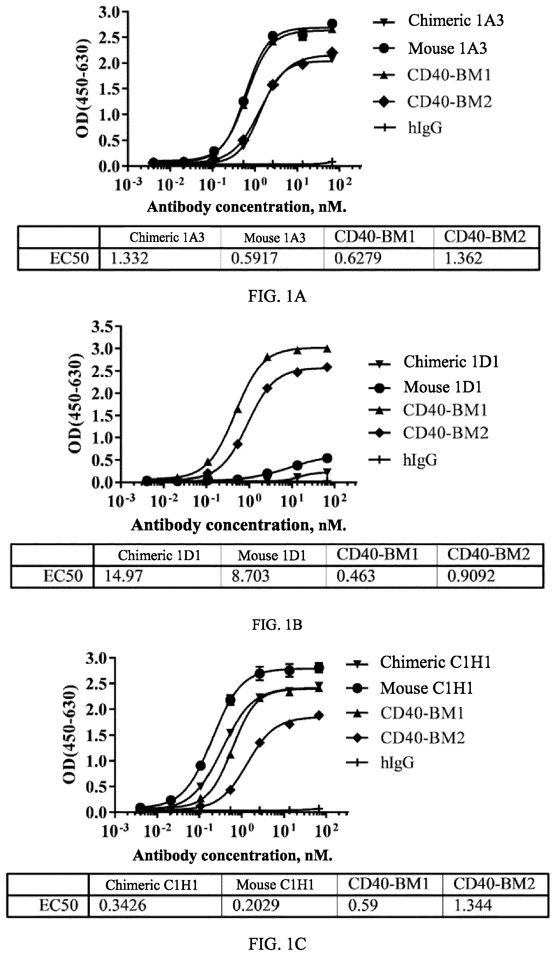

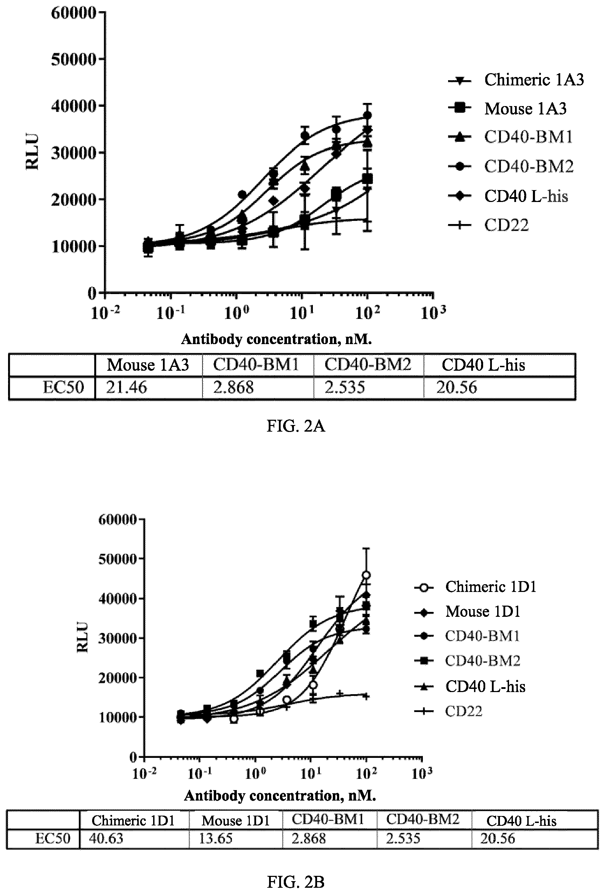

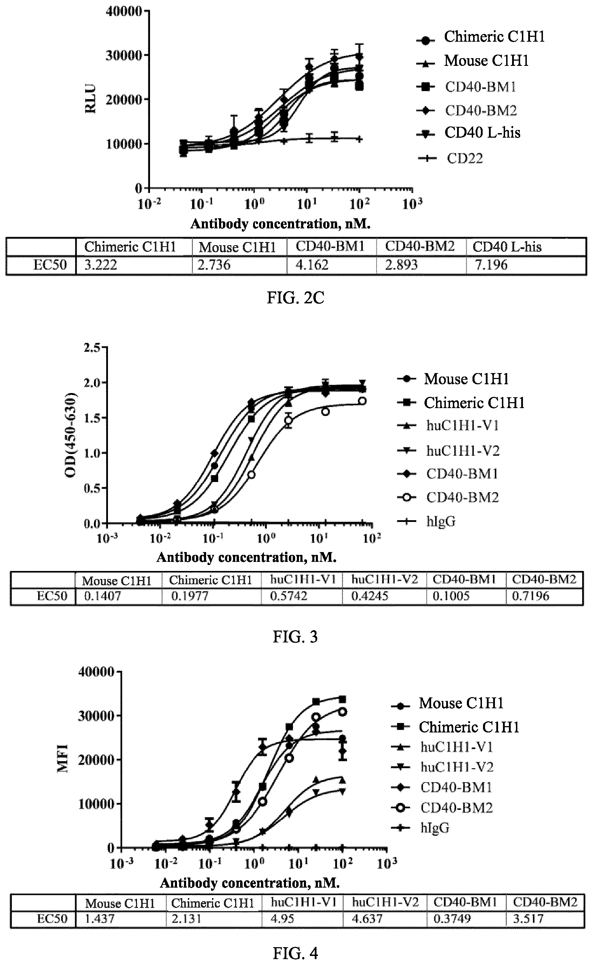

After six days of culture in a shake flask, the cell supernatant was harvested. Cells in the supernatant were collected from the precipitate of centrifugation, and the chimeric antibodies were isolated from the cell supernatant on a protein A sepharose column. The isolated chimeric antibodies were detected by capture ELISA, BIAcore affinity assay, and cell-based reporter assay as per the protocol in the previous examples. The results are shown in Table 2 and FIGs. 1A-1C and 2A-2C.

The results show that the chimeric antibodies have similar binding ability and agonistic activity to their parent antibodies. In particular, the chimeric antibody C1H1 has a higher binding affinity and ability to human CD40 and higher binding affinity to cynomolgus monkey CD40 as compared with BM1.

**Table 2. Binding affinity of chimeric antibody**

| Clone ID# | Biacore kinetics | | | | | |
|---|---|---|---|---|---|---|
| | Human CD40-his | | | Cynomolgus monkey CD40-his | | |
| | Kₐ | K_{d} | K_{D} | Kₐ | K_{d} | K_{D} |
| | (M⁻¹s⁻¹) | (s⁻¹) | (M) | (M⁻¹s⁻¹) | (s⁻¹) | (M) |
| Mouse C1H1 | 1.79E+06 | 0.001227 | 6.84E-10 | 1.39E+06 | 0.001184 | 8.52E-10 |
| Chimeric C1H1 | 1.82E+06 | 0.001567 | 8.61E-10 | 1.39E+06 | 0.001478 | 1.06E-09 |
| BM1 | 7.39E+05 | 0.006556 | 8.87E-09 | 6.23E+05 | 0.006164 | 9.89E-09 |

### Example 6 Humanization of mouse anti-CD40 monoclonal antibody C1H1

Mouse anti-CD40 antibody C1H1 was selected for humanization and further studies. The humanization of the antibody was conducted using a well-established CDR grafting method as described below.

To select a recipient frameworks for the humanization of mouse anti-CD40 antibody C1H1, the sequences of the mouse anti-CD40 antibody C1H1 light and heavy chain variable regions were blasted against the human immunoglobulin gene database. The human germline antibody with the highest homology to the mouse anti-CD40 antibody C1H1 was selected as the recipient framework for humanization. The CDRs of the heavy/light chain variable region of the mouse antibody were inserted into the selected framework and residue(s) in the framework were further mutated to give more candidate heavy/light chain variable regions. 4 humanized C1H1 antibodies, huC1H1-V1 to huC1H1-V4, were obtained, of which the heavy/light chain variable region sequences are shown in Table 1.

The vector containing the nucleotides encoding the heavy chain variable region linked to the human IgG2 heavy chain constant region (SEQ ID NO: 28) and the vector containing the nucleotides encoding the humanized light chain variable region linked to the human κ light chain constant region (SEQ ID NO: 29) were transiently transfected into 50 mL of 293F cell suspension with 1 mg/mL PEI in a light chain construct-to-heavy chain construct ratio of 60%:40%.

### Example 7 Characterization of humanized antibodies

After six days of culture in a shake flask, the cell supernatant containing the humanized antibodies huC1H1-V1 to huC1H1-V4 was harvested and the binding affinity of the antibodies to human CD40 was determined on an Octet system (Fortebio, Octet RED 96) according to the following procedures. Briefly, an anti-human IgG Fc capture (AHC) biosensor (from ForteBio) was pre-soaked in a 10 mM glycine solution (pH 1.5) for 3 s, and then soaked in a running buffer (PBST containing 0.5% w/v of BSA) for 3 s. The pre-soaking and soaking procedures were repeated 3 times. Then, the sensor was soaked in the cell supernatant containing the humanized anti-CD40 antibody, chimeric antibody C1H1 dissolved in HBS-EP⁺ buffer at a concentration of 5 µg/mL, or the benchmark dissolved in HBS-EP⁺ buffer at a concentration of 5 µg/mL for 120 s, and then soaked in the running buffer for 5 min. The new baseline was run in the running buffer for 180 s. The sensor was then soaked for 120 s in human CD40-his protein (Aero biosystems, Cat. No. CD0-H5228) 2-fold serially diluted from 40 nM in the running buffer and for 10 min in the running buffer to establish the baseline. Finally, the sensor was pre-soaked in a 10 mM glycine solution (pH 1.5) for 3 s, and then soaked in the running buffer for 3 s. The pre-soaking and soaking procedures were repeated 3 times. The association and dissociation curves were fitted to a 1:1 Langmuir binding model using ForteBio Data Analysis 8.1. Kₐ, K_{d}, and K_{D} values were determined and summarized in Table 3 below.

The results show that huC1H1-V1 and huC1H1-V2 have similar human CD40 binding affinity to the chimeric antibody C1H1 and higher binding affinity than BM2.

**Table 3. Affinity of humanized C1H1 monoclonal antibodies**

| Octet kinetics of humanized C1H1 monoclonal antibodies binding to human CD40 | | | |
|---|---|---|---|
| Clone ID# | Octet kinetics | | |
| | Human CD40-his | | |
| | Kₐ | K_{d} | K_{D} |
| | (M⁻¹s⁻¹) | (s⁻¹) | (M) |
| Chimeric C1H1 | 1.47E+06 | 9.29E-04 | 6.33E-10 |
| huC1H1-V1 | 1.41E+06 | 1.28E-03 | 9.04E-10 |
| huC1H1-V2 | 1.65E+06 | 9.81E-04 | 5.96E-10 |
| huC1H1-V3 | 5.23E+05 | 2.83E-03 | 5.41E-09 |
| huC1H1-V4 | 4.24E+05 | 3.67E-03 | 8.66E-09 |
| BM2 | 8.74E+05 | 9.79E-04 | 1.12E-09 |

The humanized antibodies huC1H1-V1 and huC1H1-V2 were purified on a protein A sepharose column, and characterized by Biacore, capture ELISA, cell-based binding FACS, competitive ELISA, cell-based reporter assay, and protein thermal shift assay according to the procedures in the previous examples and procedures described below.

A thermal stability assay was also conducted on humanized antibody huC1H1-V2. Tm (melting temperature) was determined by protein thermal shift assay using the GloMelt^{™} thermal shift protein stability kit (Biotium, Cat. No. 33022-T). Briefly, the GloMelt^{™} dye was thawed to room temperature. The vial containing the dye was vortexed and centrifuged. Then, 5 µL of the 200× dye solution was added to 95 µL of PBS to prepare a 10× dye solution. 2 µL of the 10× dye solution and 10 µg of the humanized antibody were added to the reaction system, and PBS was added to bring the total reaction volume to 20 µL. The centrifuge tubes containing the dye and the antibodies were centrifuged briefly and placed in a real-time PCR thermal cycler (Roche, LightCycler 480 II) in which the parameters of the Melt Curve program are shown in Table 4.

**Table 4. Parameters of Melt Curve program**

| Profile procedures | Temperature | Ramping rate | Holding Time |
|---|---|---|---|
| Initial hold | 25 °C | NA | 30 s |
| Melt curve | 25-99 °C | 0.1 °C/s | NA |

The measurement results are shown in Table 5 and FIGs. 3 to 7.

As shown in Table 5, the humanized antibodies huC1H1-V1 and huCIHI-V2 and the chimeric antibody C1H1 have comparable binding affinities to human CD40 and cynomolgus monkey CD40. In other words, huC1H1-V1 and huC1H1-V2 have higher binding affinities for human CD40 and cynomolgus monkey CD40 than those of BM1 and BM2 for human CD40 and cynomolgus monkey CD40.

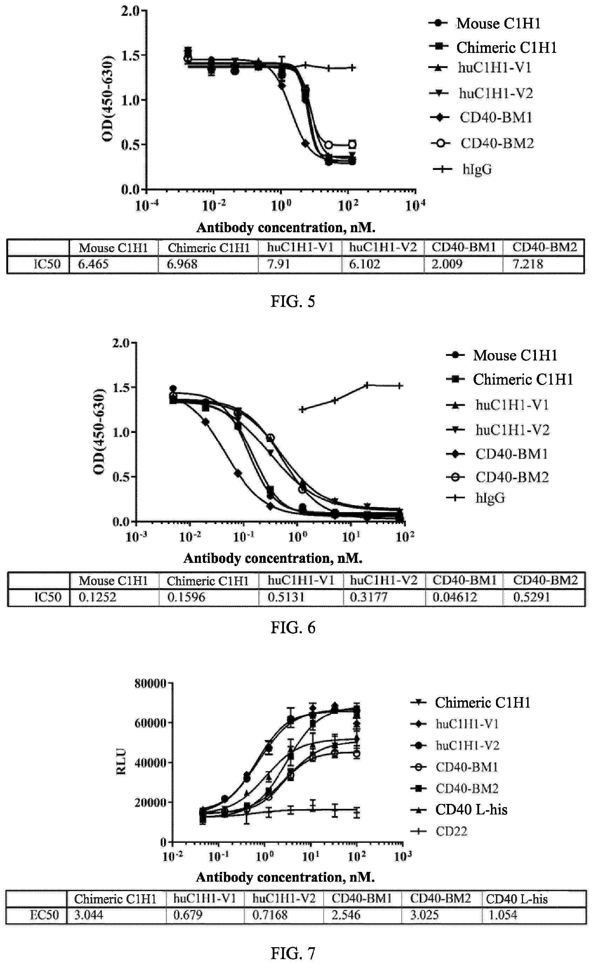

FIG. 5 shows that the humanized antibodies huC1H1-V1 and huC1H1-V2 of the present application are capable of blocking the binding of CD40 to CD40L with comparable or slightly lower blocking activity relative to BM1 and BM2.

As shown in FIG. 6, the humanized antibodies huC1H1-V1 and huC1H1-V2 of the present application were able to block the binding of human CD40 to BM2, indicating that the antibodies huC1H1-V1 and huC1H1-V2 of the present application can bind to a similar epitope to that of BM2.

As shown in FIG. 7, the agonistic activities of the humanized antibodies huC1H1-V1 and huC1H1-V2 of the present application were higher than those of BM1 and BM2 in cell-based reporter assay.

**Table 5. Binding and functional activity of humanized C1H1 antibodies**

| Biacore kinetics | | | | | | | |
|---|---|---|---|---|---|---|---|
| Clone ID# | Human CD40-his | | | Cynomolgus monkey CD40-his | | | Tm (melting temperature, °C) |
| | Kₐ | K_{d} | K_{D} | Kₐ | K_{d} | K_{D} | |
| | (M⁻¹s⁻¹) | (s⁻¹) | (M) | (M⁻¹s⁻¹) | (s⁻¹) | (M) | |
| Mouse C1H1 | 2.10E+06 | 8.97E-04 | 4.28E-10 | 2.29E+06 | 8.63E-04 | 3.77E-10 | * |
| Chimeric C1H1 | 2.51E+06 | 0.002221 | 8.84E-10 | 2.54E+06 | 0.002147 | 8.46E-10 | * |
| huC1H1-V1 | 2.25E+06 | 2.48E-03 | 1.10E-09 | 2.14E+06 | 0.002371 | 1.11E-09 | * |
| huC1H1-V2 | 2.31E+06 | 0.002704 | 1.17E-09 | 2.56E+06 | 0.002735 | 1.07E-09 | 70 |
| BM1 | 8.03E+05 | 0.007562 | 9.42E-09 | 7.64E+05 | 0.007397 | 9.68E-09 | * |
| BM2 | 2.06E+05 | 0.001616 | 7.84E-09 | 1.73E+05 | 0.001508 | 8.73E-09 | * |

| | | | | | | | |
|---|---|---|---|---|---|---|---|
| * Not tested yet. | | | | | | | |

### Example 8 In vivo anti-tumor efficacy of huC1H1-V2 antibody in human B-cell lymphoma Ramos xenograft model

The *in vivo* anti-tumor activity of antibody huC1H1-V2 was measured in NOD-SCID mice. Briefly, 3 × 10⁷ human B-cell lymphoma Ramos cells (Chinese Academy of Sciences) were injected subcutaneously in the right axilla of NOD-SCID mice. The tumor volume was measured using an electronic vernier caliper and calculated according to the formula (length × width²)/2. When the mean tumor volume reached about 100-150 mm³, 40 tumor-bearing mice were selected and randomly divided into 4 groups (10 mice per group), with the date of grouping designated as day 0. According to the administration regimen shown in Table 6, the mice were intravenously administered with the isotype control antibody (anti-HEL-Human IgG2 Isotype-control, also referred to as IgG2, Biointron Inc.) or antibody huC1H1-V2 via tail veins since day 0.

**Table 6. Design of study in human B-cell lymphoma Ramos xenograft model**

| Groups | N | Drug | Dose (mg/kg) | Route of administration | Administration volume (mL/kg) | Administration regimen |
|---|---|---|---|---|---|---|
| 1 | 10 | Isotype control antibody (IgG2) | 15 | i.v. | 10 | Q2D × 8 doses |
| 2 | 10 | huC1H1-V2 | 1.5 | i.v. | 10 | Q2D × 8 doses |
| 3 | 10 | huC1H1-V2 | 5 | i.v. | 10 | Q2D × 8 doses |
| 4 | 10 | huC1H1-V2 | 15 | i.v. | 10 | Q2D × 8 doses |

| | | | | | | |
|---|---|---|---|---|---|---|
| Note: N: number of animals in the group; Q2D: once every 2 days; iv: intravenous administration. | | | | | | |

The results are summarized in Table 7. All treatments were well tolerated in the tumor-bearing animals without significant weight loss or other symptoms. The treatment with antibody huC1H1-V2 (5 mg/kg) exhibited the strongest anti-tumor activity, with the mean tumor volume in the group (group 3) being 230.2 mm³ significantly less than that of the isotype control group and the tumor growth inhibition (TGI) being 96% on day 14. However, the treatment with antibody huC1H1-V2 (15 mg/kg) did not exhibit further enhanced efficacy. That is, no significant difference was found in tumor volume between group 4 and group 3.

**Table 7. Anti-tumor efficacy of antibody huCIHI-V2 in human B-cell lymphoma Ramos xenograft model**

| Treatment | Tumor volume on day 0 (mm³)^{a} | Tumor volume on day 14 (mm³)^{a,b} | T/C (%)^{c} | TGI (%)^{d} | P value |
|---|---|---|---|---|---|
| IgG2, 15 mg/kg, Q2D | 114.7±1.4 | 2830.4±235.7 | - | - | - |
| huC1H1-V2, 1.5 mg/kg, Q2D | 111.1±2.0 | 431.9±58.0*** | 12 | 88 | <0.001 |
| huC1H1-V2, 5 mg/kg, Q2D | 114.5±1.5 | 230.2±36.3*** | 4 | 96 | <0.001 |
| huC1H1-V2, 15 mg/kg, Q2D | 114.3±1.3 | 242.5±22.1*** | 5 | 95 | <0.001 |

| | | | | | |
|---|---|---|---|---|---|
| Note: a, mean ± standard error; b, tumor volume on day 14 as compared with the isotype control group by the Student's t test, wherein * denotes P ≤ 0.05, ** denotes P < 0.01, and *** denotes P < 0.001; c, T/C (%) = (T - T₀)/(C - C₀ × 100%, wherein T and C refer to the mean tumor volume of the treatment group and the isotype control group on day 14, respectively, and T₀ and C₀ refer to the mean tumor volume of the treatment group and the isotype control group on day 0, respectively; d, TGI (%) = (1 - T/C) × 100%. | | | | | |

### Example 9 In vivo anti-tumor efficacy of huC1H1-V2 antibody in colon carcinoma MC38 xenograft mouse model

The *in vivo* anti-tumor activity of antibody huC1H1-V2 was further determined in humanized CD40 transgenic mice (also referred to as hCD40 mice). Briefly, 1 × 10⁶ mouse colon carcinoma MC38 cells (Shanghai Lanli Biotech, LTD., CO.) were injected subcutaneously on the right back of hCD40 mice. The tumor volume was measured using an electronic vernier caliper and calculated according to the formula (length × width²)/2. When the mean tumor volume reached about 100 mm³, 18 tumor-bearing mice were selected and randomly divided into 3 groups (6 mice per group), with the date of grouping designated as day 0. According to the administration regimen shown in Table 8, the mice were intravenously administered with the solvent (phosphate buffered saline, or PBS) or antibody huC1H1-V2 via tail veins since day 1.

**Table 8. Design of study in MC38 xenograft model**

| Groups | N | Drug | Dose (mg/kg) | Route of administration | Administration volume (mL/kg) | Administration regimen |
|---|---|---|---|---|---|---|
| 1 | 6 | Solvent (PBS) | - | i.v. | 10 | Q2D × 7 doses |
| 2 | 6 | huC1H1-V2 | 3 | i.v. | 10 | Q2D × 7 doses |
| 3 | 6 | huC1H1-V2 | 10 | i.v. | 10 | Q2D × 7 doses |

| | | | | | | |
|---|---|---|---|---|---|---|
| Note: N: number of animals in the group; Q2D: once every 2 days; iv: intravenous administration. | | | | | | |

The results are summarized in Table 9. All treatments were well tolerated in the tumor-bearing animals without significant weight loss or any symptoms. The treatment with antibody huC1H1-V2 (3 mg/kg) exhibited significant anti-tumor activity, with the mean tumor volume in the group (group 2) being 1169 mm³ significantly less than that of the solvent group and the tumor growth inhibition (TGI) being 64% on day 14. The treatment with antibody huC1H1-V2 (10 mg/kg) also exhibited significant anti-tumor activity, with the mean tumor volume in the group (group 3) being 1446 mm³ significantly less than that of the solvent group and the tumor growth inhibition (TGI) being 56% on day 14.

**Table 9. Anti-tumor efficacy of antibody huC1H1-V2 in MC38 xenograft model**

| Treatment | Tumor volume on day 0 (mm³)^{a} | Tumor volume on day 14 (mm³)^{a,b} | T/C (%)^{c} | TGI (%)^{d} | P value |
|---|---|---|---|---|---|
| Solvent (PBS), Q2D | 112±9 | 3241±383 | - | - | - |
| huC1H1-V2, 3 mg/kg, Q2D | 111±5 | 1169±217** | 36 | 64 | <0.01 |
| huC1H1-V2, 10 mg/kg, Q2D | 113±8 | 1446±467* | 44 | 56 | 0.014 |

| | | | | | |
|---|---|---|---|---|---|
| Note: a, mean ± standard error; b, tumor volume on day 14 as compared with the solvent group by the Student's t test, wherein * denotes P ≤ 0.05, and ** denotes P < 0.01; c, T/C (%) = T_{RTV}/C_{RTV} × 100%, wherein T_{RTV} and C_{RTV} refer to the mean RTV of the treatment group and the control group, respectively, and RTV = Vₜ/V₀, wherein Vₜ refers to the mean tumor volume on day 14 and V₀ refers to the mean tumor volume on day 0; d, TGI (%) = (1 - T/C) × 100%. | | | | | |

### Example 10 Efficacy of anti-CD40 antibody and anlotinib or pharmaceutically acceptable salt thereof in human B-cell lymphoma Ramos subcutaneous xenograft mouse model

Human B-cell lymphoma Ramos cells (purchased from the Cell Bank of the Chinese Academy of Sciences) were cultured in an RPMI1640 medium containing 10% of fetal bovine serum and penicillin-streptomycin. The cells were inoculated in a 37 °C/5% CO₂ incubator until the logarithmic growth phase and collected for grafting.

The mice used in this study were NOD-Scid mice (purchased from Shanghai Lingchang Biotechnology Co., Ltd.) aged about 6 weeks and bred in specific pathogen-free conditions in 12/12-hour light/dark cycles. The health status of the animals was monitored daily.

Each mouse was inoculated subcutaneously with 1 × 10⁷ Ramos cells. When the tumor grew to 80-100 mm³, the mice were divided into groups of 10 according to the tumor volume. The administration regimen is shown in Table 10, with the first dosing day designated as D0. Antibody huC1H1-V2 and IgG2 (Biointron, Cat. No. B107803) were both formulated to desired concentrations with normal saline, and anlotinib dihydrochloride was formulated at desired concentrations with distilled water. At the end of study (D14), the animals were sacrificed by CO₂ anesthesia and dissected to give the tumors. The tumors were photographed.

The tumor diameters were measured twice weekly with a vernier caliper. Tumor volume (V) = 1/2 × a × b², wherein a and b denote length and width, respectively; T/C (%) = (T - T0)/(C - C0) × 100, wherein T and C refer to the tumor volumes of the treatment group and the IgG2 control group on day 14, respectively, and T0 and C0 refer to the tumor volumes of the treatment group and IgG2 control group on day 0, respectively; the tumor growth inhibition% (TGI%) = 100 - T/C (%). When the tumor regressed, the tumor growth inhibition% (TGI%) = 100 - (T - T0)/T0 × 100. If the tumor volume was smaller than the initial volume, i.e., T < T0 or C < C0, the outcome was defined as partial regression (PR); if the tumors completely disappeared, i.e., the outcome was defined as complete regression (CR).

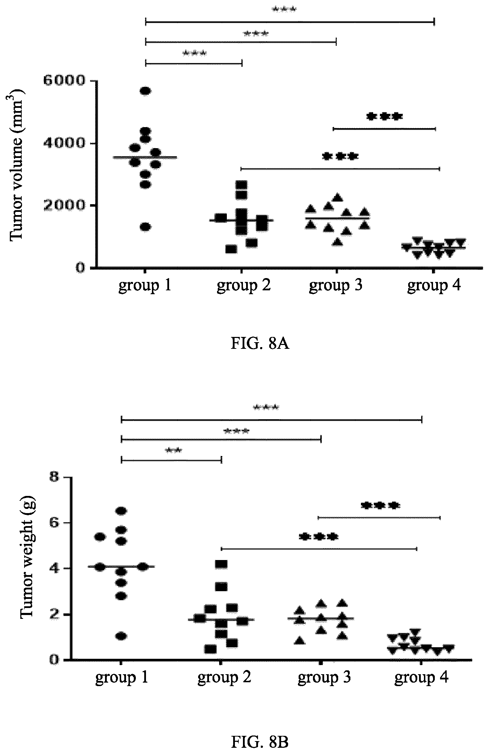

As shown in Table 11 and FIGs. 8A (tumor volume) and 8B (tumor weight), on day 14, anti-CD40 antibody huCIHI-V2 (0.5 mg/kg) and the dihydrochloride of anlotinib (1 mg/kg) both exhibited a significant inhibitory effect on the growth of the subcutaneous human B-cell lymphoma Ramos tumor graft in mice, with tumor growth inhibition rates being 58% and 56%, respectively; in terms of the combined use, the tumor growth inhibition rate was improved to 84%; the tumor volume and tumor weight in group 4 were significantly lower than those of groups 2 and 3 (P < 0.001). The above drugs were well tolerated in the tumor-bearing mice, with no significant symptoms such as weight loss, suggesting that the combination of huC1H1-V2 and anlotinib dihydrochloride has a significant synergistic effect and no increased toxicity.

**Table 10. Administration regimen**

| Groups | Name and dose of treatment | Administration volume | Route of administration | Treatment cycle and frequency |
|---|---|---|---|---|
| 1 | 0.5mg/kg IgG2 | 0.1mL/10g | Intraperitoneal administration | Once every 2 days, 8 doses |
| 2 | 0.5mg/kg huC1H1-V2 | 0.1mL/10g | Intraperitoneal administration | Once every 2 days, 8 doses |
| 3 | Anlotinib dihydrochloride, 1 mg/kg | 0.1mL/10g | Intragastric administration | Once every day, 15 doses |
| 4 | huC1H1-V2, 0.5 mg/kg + Anlotinib dihydrochloride, 1 mg/kg | 0.1mL/10g | Intraperitoneal administration + Intragastric administration | Once every 2 days, 8 doses + Once every day, 15 doses |

| | | | | |
|---|---|---|---|---|
| Note: in group 4, 0.5 mg/kg of huC1H1-V2 was intraperitoneally administered once every 2 days for 8 doses; 1 mg/kg of anlotinib dihydrochloride was intragastrically administered once every day for 15 doses. | | | | |

**Table 11. Efficacy in human B-cell lymphoma Ramos subcutaneous xenograft mouse model**

| Groups | Mean tumor volume (mm³) (mean±SD) | | T/C (%) | TGI (%) | P value | Number of animals per group at the start of study | Number of animals per group at the start of study |
|---|---|---|---|---|---|---|---|
| | D0 | D14 | D14 | | | | |
| 1 | 90.4±0.9 | 3554.3±361.8 | - | - | - | 10 | 10 |
| 2 | 89.3±0.8 | 1546.7±198.5 | 42 | 58*** | <0.001 | 10 | 10 |
| 3 | 85.3±3.8 | 1602.8±137.1 | 44 | 56*** | <0.001 | 10 | 10 |
| 4 | 89.9±0.6 | 639.0±54.7 | 16 | 84 | <0.001 | 10 | 10 |

| | | | | | | | |
|---|---|---|---|---|---|---|---|
| Note: P values were obtained from tumor volume comparison with the IgG2 group by the Mann-Whitney test; *** denotes p < 0.001 based on the tumor volume comparison with the huC1H1-V2 + anlotinib dihydrochloride group by the two-tailed Student's t assay; p < 0.05 was defined as a statistically significant difference. | | | | | | | |

The sequence information of the present application is summarized in Table 12 below.

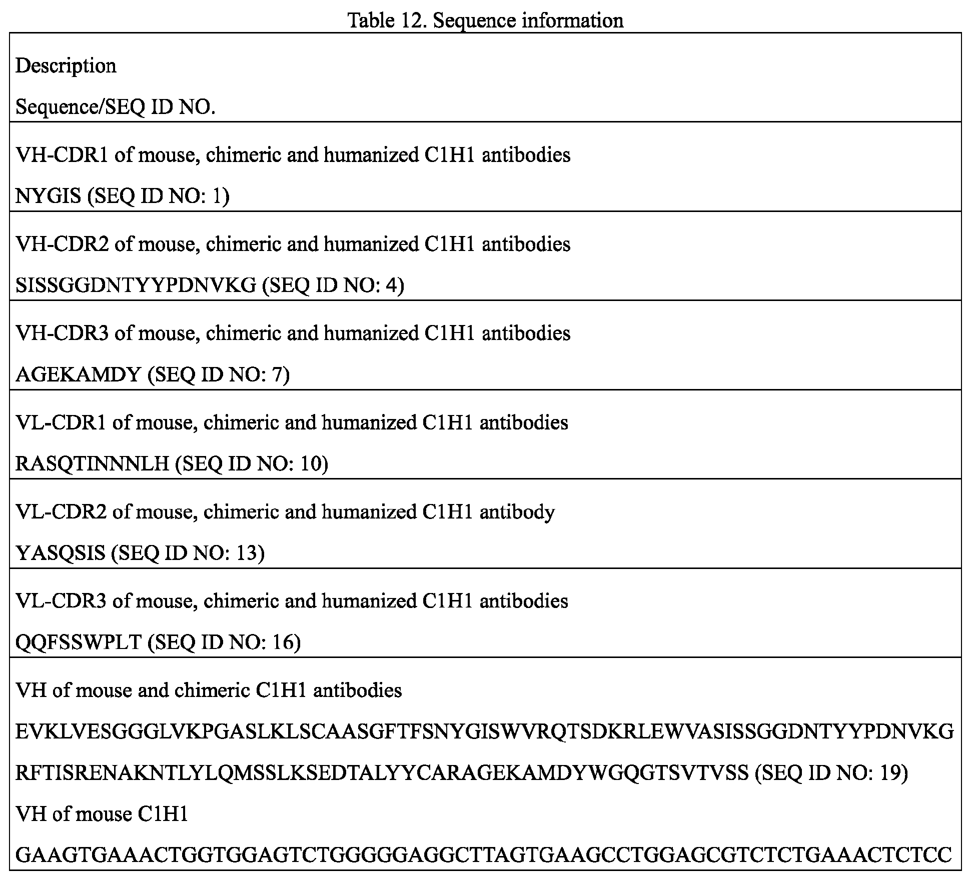

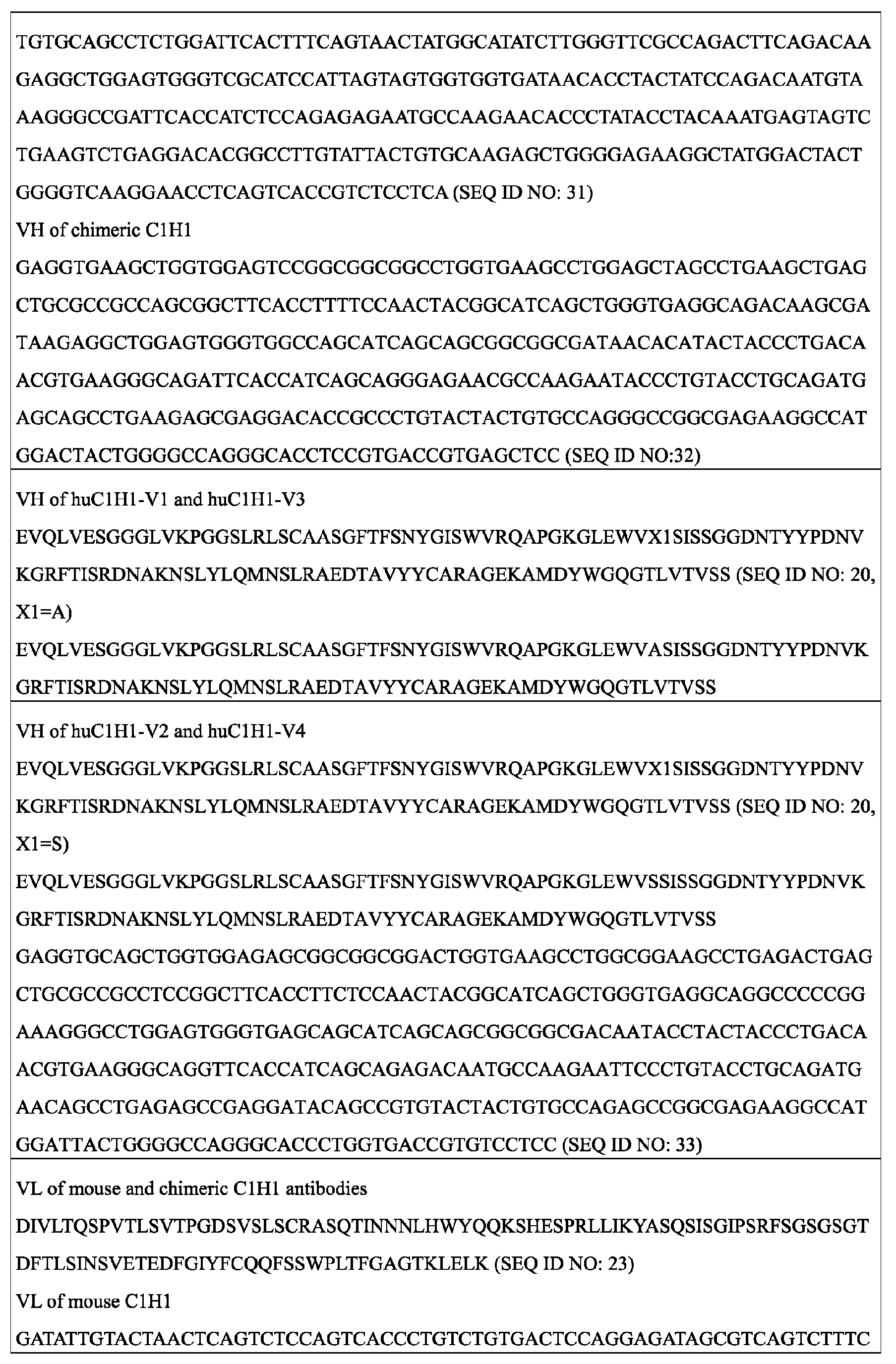

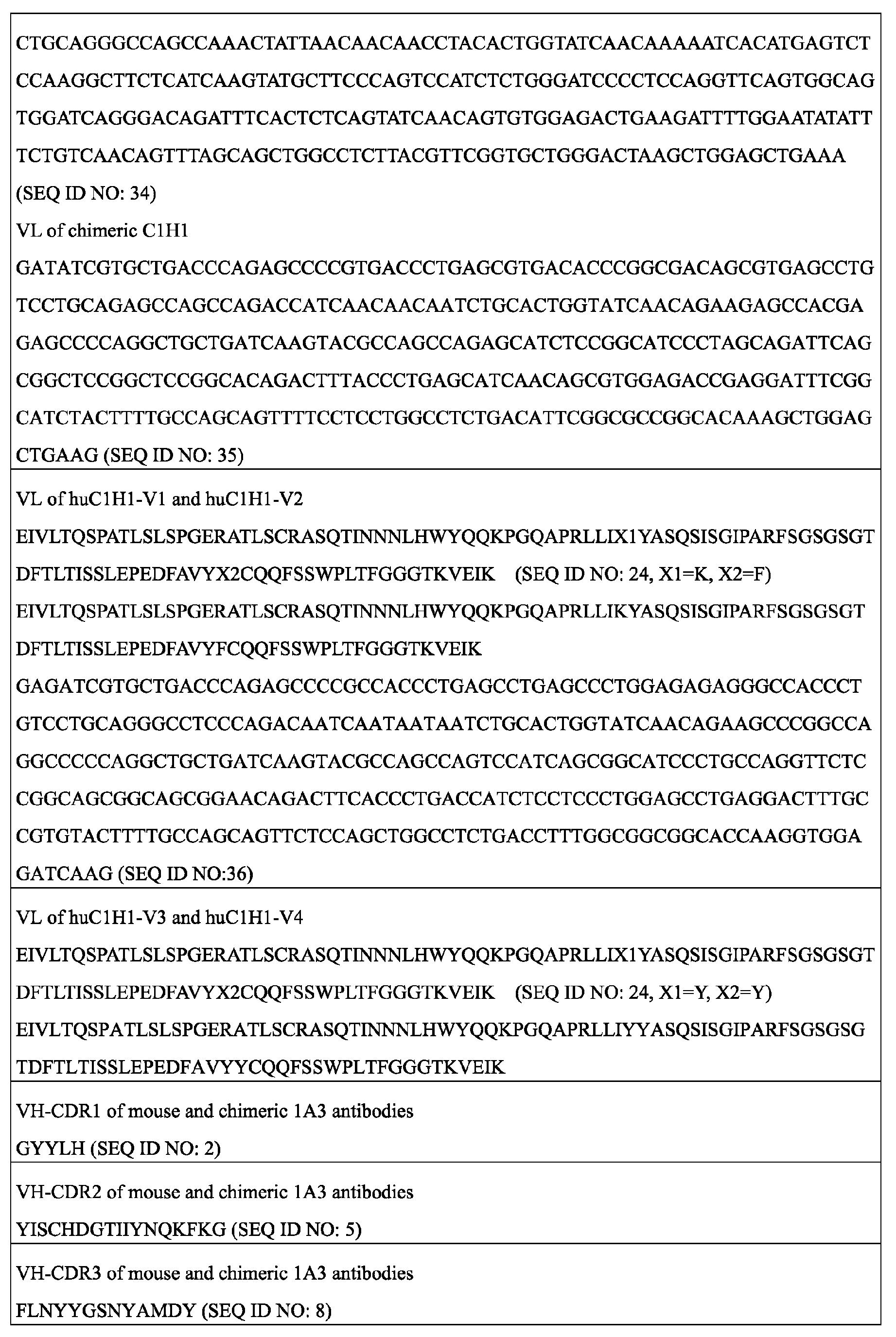

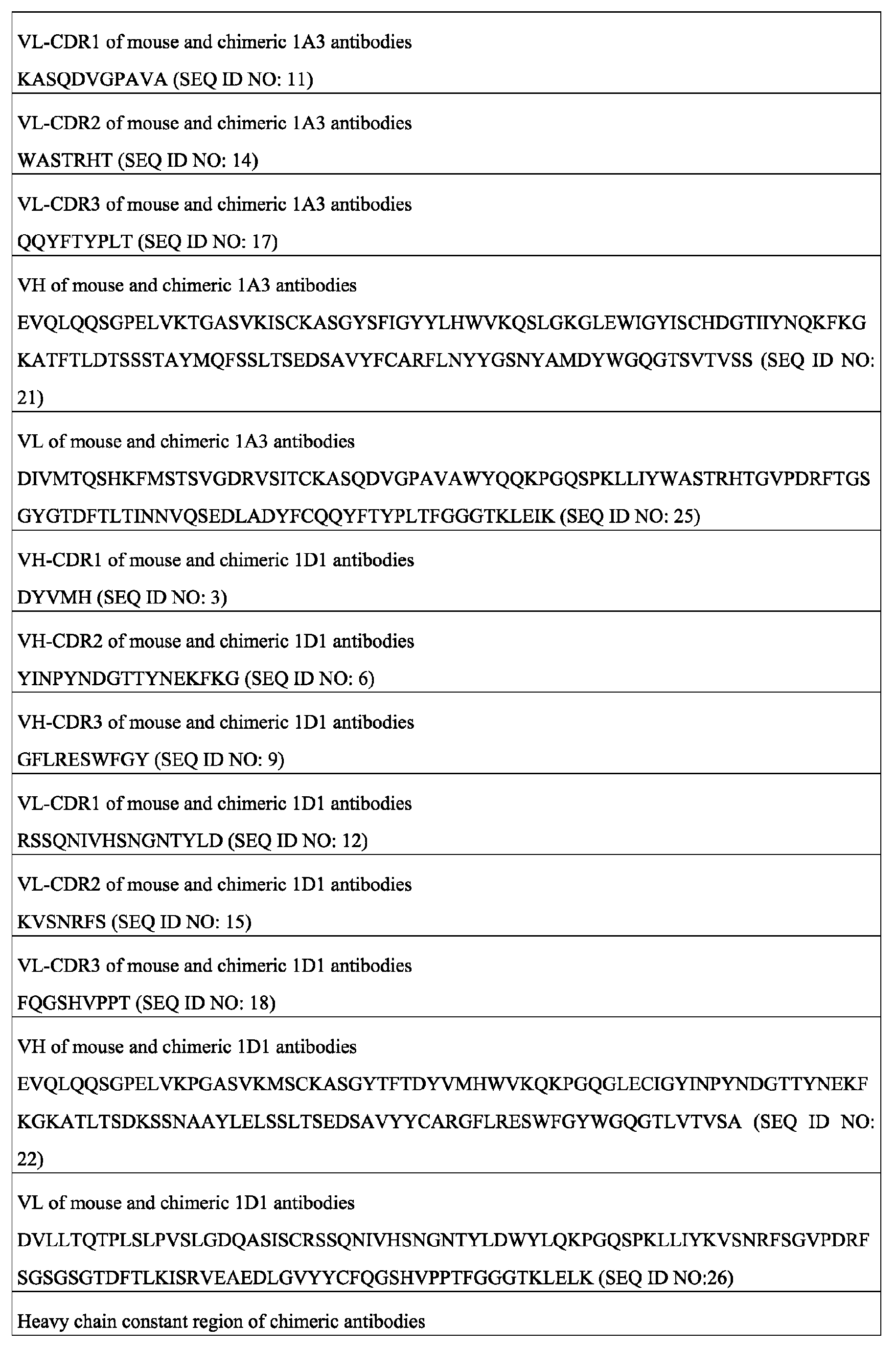

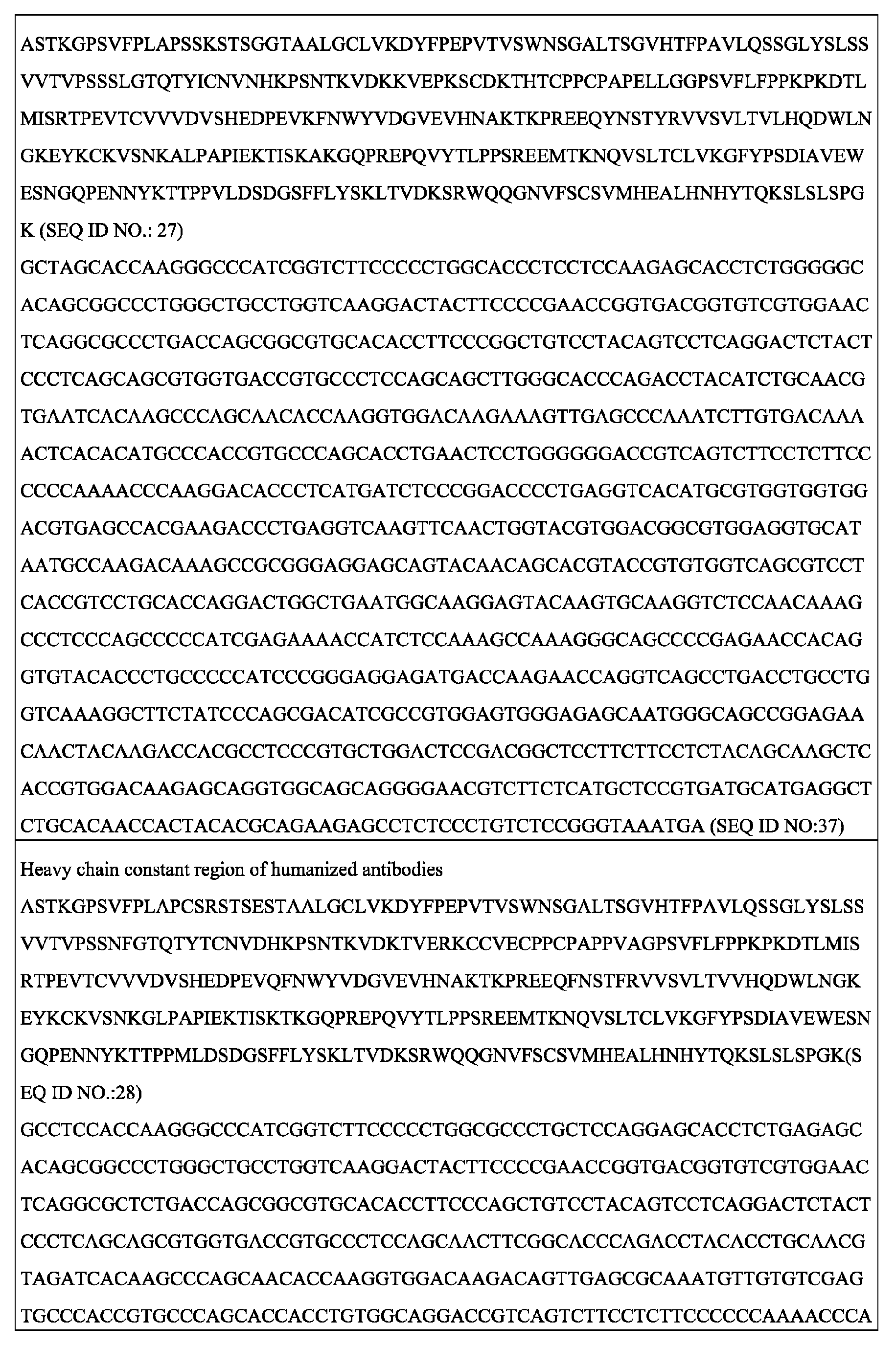

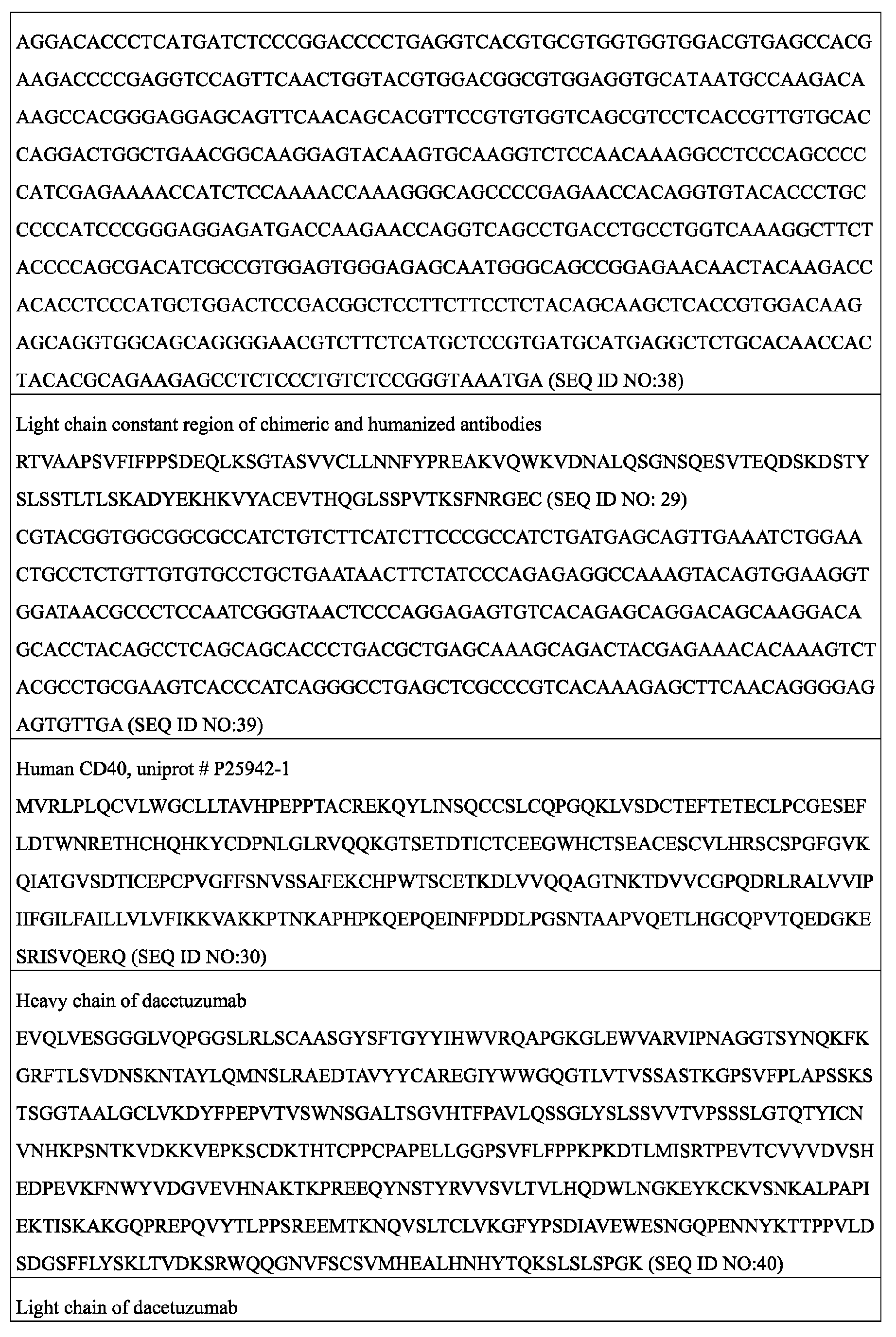

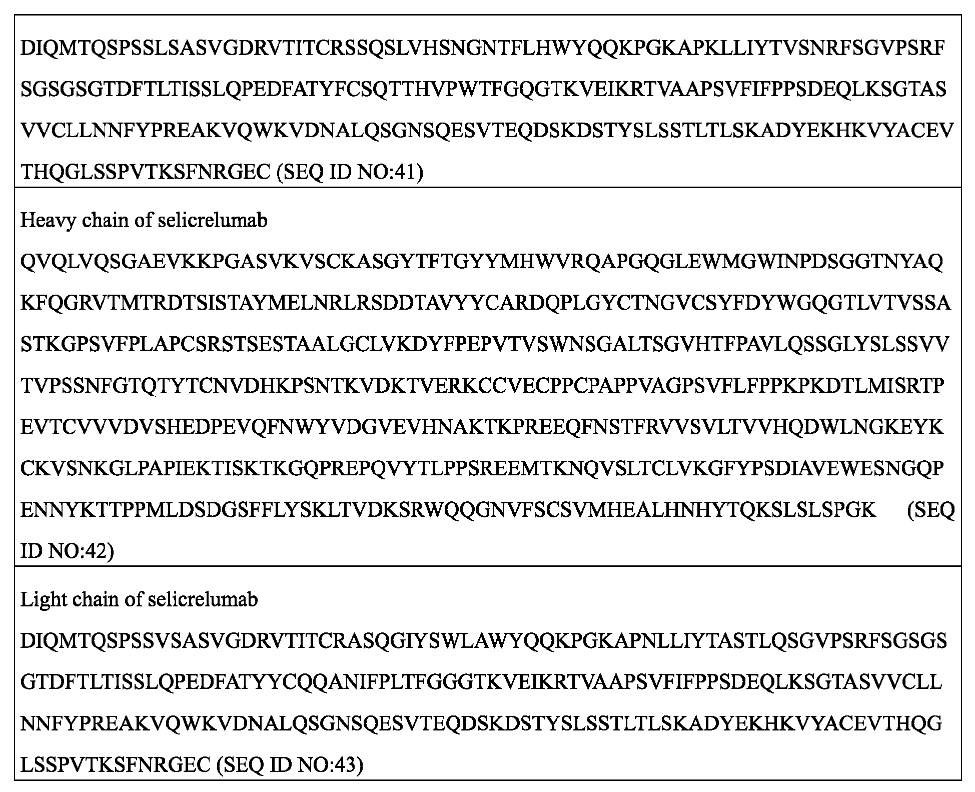

Although the present disclosure has been described in conjunction with one or more embodiments, it will be appreciated that the present disclosure is not limited to such embodiments, but is intended to encompass all alternatives, modifications, and equivalents included within the spirit and scope of the appended claims. All the references cited herein are incorporated by reference in their entireties.

## Claims

1. A pharmaceutical combination, comprising: (a) an anti-CD40 antibody or an antigen-binding portion thereof, and (b) a second therapeutic agent, wherein the second therapeutic agent is a tyrosine kinase inhibitor.

2. The pharmaceutical combination according to claim 1, wherein the second therapeutic agent is a VEGFR tyrosine kinase inhibitor, the VEGFR tyrosine kinase inhibitors including anlotinib, vandetanib, sorafenib, cediranib, vatalanib, pazopanib, motesanib, lenvatinib, sunitinib, bevacizumab, ramucirumab, or a pharmaceutically acceptable salt thereof.

3. The pharmaceutical combination according to claim 1 or 2, wherein the second therapeutic agent is anlotinib or a pharmaceutically acceptable salt thereof; optionally, the pharmaceutically acceptable salt of anlotinib is hydrochloride or dihydrochloride.

4. The pharmaceutical combination according to any one of claims 1-3, wherein the anti-CD40 antibody or the antigen-binding portion thereof comprises a heavy chain variable region and a light chain variable region; the heavy chain variable region comprises a V_{H} CDR1, a V_{H} CDR2, and a V_{H} CDR3, and the light chain variable region comprises a V_{L} CDR1, a V_{L} CDR2, and a V_{L} CDR3, wherein (1) the V_{H} CDR1, V_{H} CDR2, V_{H} CDR3, V_{L} CDR1, V_{L} CDR2, and V_{L} CDR3 comprise amino acid sequences having at least 85% identity to the amino acid sequences set forth in SEQ ID NOs: 1, 4, 7, 10, 13, and 16, respectively; (2) the V_{H} CDR1, V_{H} CDR2, V_{H} CDR3, V_{L} CDR1, V_{L} CDR2, and V_{L} CDR3 comprise amino acid sequences having at least 85% identity to the amino acid sequences set forth in SEQ ID NOs: 2, 5, 8, 11, 14, and 17, respectively; or (3) the V_{H} CDR1, V_{H} CDR2, V_{H} CDR3, V_{L} CDR1, V_{L} CDR2, and V_{L} CDR3 comprise amino acid sequences having at least 85% identity to the amino acid sequences set forth in SEQ ID NOs: 3, 6, 9, 12, 15, and 18, respectively.

5. The pharmaceutical combination according to any one of claims 1-4, wherein the anti-CD40 antibody or the antigen-binding portion thereof comprises a heavy chain variable region, the heavy chain variable region comprising an amino acid sequence having at least 85% identity to the amino acid sequence set forth in SEQ ID NO: 19, 20, 21, or 22, wherein in SEQ ID NO: 20, X1 = A or S.

6. The pharmaceutical combination according to any one of claims 1-5, wherein the anti-CD40 antibody or the antigen-binding portion thereof comprises a light chain variable region, the light chain variable region comprising an amino acid sequence having at least 85% identity to the amino acid sequence set forth in SEQ ID NO: 23, 24, 25, or 26, wherein in SEQ ID NO: 24, X1 = K and X2 = F, or X1 = Y and X2 = Y

7. The pharmaceutical combination according to any one of claims 1-6, wherein the anti-CD40 antibody or the antigen-binding portion thereof comprises a heavy chain variable region and a light chain variable region, the heavy chain variable region and the light chain variable region comprise: (1) amino acid sequences having at least 85% identity to the amino acid sequences set forth in SEQ ID NOs: 19 and 23, respectively; (2) amino acid sequences having at least 85% identity to the amino acid sequences set forth in SEQ ID NOs: 20 and 24, respectively, wherein in SEQ ID NO: 20, X1 = A, and in SEQ ID NO: 24, X1 = K and X2 = F; (3) amino acid sequences having at least 85% identity to the amino acid sequences set forth in SEQ ID NOs: 20 and 24, respectively, wherein in SEQ ID NO: 20, X1 = S, and in SEQ ID NO: 24, X1 = K and X2 = F; (4) amino acid sequences having at least 85% identity to the amino acid sequences set forth in SEQ ID NOs: 20 and 24, respectively, wherein in SEQ ID NO: 20, X1 = A, and in SEQ ID NO: 24, X1 = Y and X2 = Y; (5) amino acid sequences having at least 85% identity to the amino acid sequences set forth in SEQ ID NOs: 20 and 24, respectively, wherein in SEQ ID NO: 20, X1 = S, and in SEQ ID NO: 24, X1 = Y and X2 = Y; (6) amino acid sequences having at least 85% identity to the amino acid sequences set forth in SEQ ID NOs: 21 and 25, respectively; or (7) amino acid sequences having at least 85% identity to the amino acid sequences set forth in SEQ ID NOs: 22 and 26, respectively.

8. The pharmaceutical combination according to any one of claims 4-7, wherein the anti-CD40 antibody or the antigen-binding portion thereof comprises a heavy chain constant region linked to the heavy chain variable region and a light chain constant region linked to the light chain variable region, and the heavy chain constant region comprises the amino acid sequence set forth in SEQ ID NO: 27 or 28, and the light chain constant region comprises the amino acid sequence set forth in SEQ ID NO: 29.

9. The pharmaceutical combination according to any one of claims 1-8, wherein the pharmaceutical combination further comprises a third therapeutic agent.

10. The pharmaceutical combination according to claim 9, wherein the third therapeutic agent comprises a chemotherapeutic drug; optionally, the chemotherapeutic drug comprises one or more of alkylating agents, podophyllum drugs, platinum-based drugs, fluoropyrimidine derivatives, cytosine derivatives, taxane drugs, camptothecin analog drugs, anthracycline drugs, and vinca alkaloid drugs.

11. A kit comprising the pharmaceutical combination according to any one of claims 1-10.

12. Use of the pharmaceutical combination according to any one of claims 1-10 or the kit according to claim 11 for preparing a medicament for treating cancer.

13. The use according to claim 12, wherein the anti-CD40 antibody or the antigen-binding portion thereof and the second therapeutic agent are each present in the form of a formulation, which can be administered simultaneously, sequentially, and/or alternately; optionally, the anti-CD40 antibody or the antigen-binding portion thereof, the second therapeutic agent and the third therapeutic agent are each present in the form of a formulation, which can be administered simultaneously, sequentially, and/or alternately.

14. The use according to claim 12 or 13, wherein the cancer is a solid tumor; optionally, the solid tumor includes skin cancer, pancreatic cancer, colorectal cancer, esophageal cancer, gastrointestinal cancer, prostate cancer, bladder cancer, kidney cancer, ovarian cancer, uterine cancer, breast cancer, lung cancer, thyroid cancer, nasopharyngeal carcinoma, and liver cancer.

15. The use according to claim 12 or 13, wherein the cancer is a B cell malignancy; optionally, the B cell malignancy includes non-Hodgkin's lymphoma, chronic lymphocytic leukemia, multiple myeloma, B cell lymphoma, high-grade B cell lymphoma, medium-grade B cell lymphoma, low-grade B cell lymphoma, B cell acute lymphocytic leukemia, Hodgkin's lymphoma, plasmacytoma, follicular lymphoma, follicular small cleaved cell lymphoma, follicular large cell lymphoma, follicular mixed small cleaved cell lymphoma, diffuse small cleaved cell lymphoma, small lymphocytic lymphoma, prolymphocytic leukemia, lymphoplasmacytic lymphoma, marginal zone lymphoma, mucosa-associated lymphoid tissue lymphoma, monocytic B cell lymphoma, splenic leukemia, hairy cell leukemia, diffuse large B cell lymphoma, mediastinal large B cell lymphoma, lymphomatoid granulomatosis, intravascular lymphoma, mixed lymphoma, immunoblastic lymphoma, Burkitt lymphoma, AIDS-related lymphoma, Waldenstrom's macroglobulinemia, and mantle cell lymphoma; optionally, the cancer is a non-B cell hematological malignancy; optionally, the non-B cell hematological malignancies includes chronic myeloid leukemia and acute myeloid leukemia.
